(19) Europäisches Patentamt
European Patent Office
Office européen des brevets

(11) Publication number: 0 110 633 A2

# (12) EUROPEAN PATENT APPLICATION

(21) Application number: 83307032.9

(22) Date of filing: 17.11.83

(51) Int. Cl.³: G 03 C 7/26, G 03 C 7/32

(30) Priority: 17.11.82 JP 202452/82

(43) Date of publication of application: 13.06.84 Bulletin 84/24

(84) Designated Contracting States: DE FR GB

(71) Applicant: KONISHIROKU PHOTO INDUSTRY CO. LTD., No. 26-2, Nishishinjuku 1-chome Shinjuku-ku, Tokyo 160 (JP)

(72) Inventor: Kida, Shuji, 2-5-19, Owada-cho, Hachioji-shi Tokyo (JP)
Inventor: Nakagawa, Satoshi, 294-6, Kamikuzawa, Sagamihara-shi Kanagawa-ken (JP)
Inventor: Kawashima, Yasuhiko, 3-9-22 Azuma-cho, Akishima-shi Tokyo (JP)
Inventor: Masuda, Kosaku, 5-16-2-106 Tamagawa-cho, Akishima-shi Tokyo (JP)

(74) Representative: Ellis-Jones, Patrick George Armine et al, J.A. KEMP & CO. 14 South Square Gray's Inn, London WC1R 5EU (GB)

## (54) Silver halide photographic light-sensitive material.

(57) A silver halide photographic light-sensitive material is described which comprises a support having thereon a plurality of silver halide emulsion layers which possess different speeds but sensitivities in the same spectral region and at least one light-sensitive layer comprising a non-diffusible coupler capable of forming non-diffusible dye by reaction with an oxidation product of a color developing agent, wherein the silver halide emulsion layer having the highest speed among said plurality of silver halide emulsion layers contains a non-diffusible coupler capable of producing a movable dye by reaction thereof with an oxidation product of a color developing agent and a non-diffusible compound capable of releasing a development inhibitor by reaction thereof with an oxidation product of a color developing agent.

EP 0 110 633 A2

# SILVER HALIDE PHOTOGRAPHIC LIGHT-SENSITIVE MATERIAL

## DETAILED DESCRIPTION OF THE INVENTION

The present invention relates to a silver halide photographic light-sensitive material, and more particularly to a silver halide photographic light-sensitive material having improved graininess and excellent sharpness.

It has hitherto been known that DIR compounds may be used as one of the techniques for improving the graininess and sharpness of photographic images. The above DIR compound reacts with the oxidized product of a color developing agent to release imagewise a development inhibitor to inhibit the silver development, thereby preventing possible enormous growth of dye cloud and improving the graininess. This technique was very useful means for such light-sensitive materials as 110 size or 35mm size films, but for such films of the further smaller image frame size as the recent disc film, because it requires a much larger magnification in making enlarged photographic prints, so that the use of the above DIR compound

alone is not enough for improving the image quality, and thus coarse graininess becomes conspicuous in the resulting pints.

In contrast to this, a method for improving the graininess by use of a nondiffusible coupler capable of producing a movable dye is recently disclosed in Japanese Patent Publication Open to Public Inspection (hereinafter referred to as Japanese Patent O.P.I. Publication) No.82837/1982. The technique in this method, as described in the publication, shows an improving effect upon the graininess but does not come up to the improvement on the sharpness. The above technique is therefore desirable to be utilized in a layer for producing an yellow dye, to which color the naked eye is less sensitive, so that the technique lacks the actual improvement effect on the graininess of a photographic image that is easily distinguishable by the naked eye.

It is therefore an object of the present invention to provide a silver halide photographic light-sensitive material which is capable of making up for the above disadvantages, and of forming a photographic image excellent not only in the graininess but also in the sharpness.

As a result of our continued various investigations, it has now been found that the above object can be accomplished by the following silver halide photographic light-sensitive material comprising a support having thereon plural silver halide emulsion layer which are respectively different in

speed but have the sensitivity thereof in the same spectral region and at least one light-sensitive layer comprising a non-diffusible coupler capable of forming non-diffusible dye by the reaction thereof with a oxidized product of a color developing agent, wherein the silver halide emulsion layer having highest speed among said plural silver halide emulsion layers contains a non-diffusible coupler capable of producing a movable dye by the reaction thereof with the oxidized product of a color developing agent and a non--diffusible compound capable of releasing a development by the reaction thereof with the oxidized product of a color developing agent.

Namely, the present invention is characterized by such a silver halide photographic light-sensitive material as comprising not less than two emulsion layers differing in the speed but having the sensitivity thereof to the same spectral region and containing a nondiffusible dye-formable nondiffusible coupler, of which emulsion layers the highest speed-having silver halide emulsion layer contains a movable dye producible nondiffusible coupler and a development inhibitor-releasable nondiffusible compound. And by the use of such a light-sensitive material as having the above construction, even in the case of miniaturized film like the previously mentioned disc film, not only the graininess but also the sharpness can be improved.

The present invention will be illustrated further in detail below:

The light-sensitive material of the present invention, if it is of a multilayer construction, is prepared by coating on a support in order from the support side red-sensitive, green-sensitive and blue-sensitive emulsion layers, and when dividing one of these layers or all the layers each into silver halide emulsion layers differing in the speed, the layer closer to the support is made to be the lower layer.

The aforesaid nondiffusible coupler capable of producing a movable dye by the reaction thereof with the oxidized product of a color developing agent and contained in the highest-speed emulsion layer of the silver halide photographic light-sensitive material constructed like above according to the present invention can be broadly classified into two types of compounds: one is a group of slightly movable dye-producible nondiffusible couplers and the other a group of completely diffusible dye-producible nondiffusible couplers.

The expression, "slightly movable" used herein means being movable within the layer containing the movable dye-producible nondiffusible coupler, and the expression, "completely diffusible" means that the produced dye diffuses from the layer containing the above nondiffusible coupler to the adjacent layers.

And the effect by the former coupler is such that the

dye produced by the reaction of the coupler with the oxidized product of a color developing agent slightly moves during a processing to spread thinly the dye cloud to thereby improve the graininess, and at the same time the spread of the dye cloud is controlled to an appropriate extent by a compound which, as will be described hereinafter, releases a development inhibitor material by the above exidized product (the compound is hereinafter called "DIR compound") to thereby improve the sharpness.

The DIR compound, which will be described in detail hereinafter, as is generally known, is one that performs a coupling reaction with the oxidized product of a color developing agent during a color developing process, thereby releasing a development inhibitor material.

The thus released development inhibitor material may be a compound that spreads at least into not only the DIR compound-containing emulsion layer but also the layer adjacent thereto and belongs to the so-called diffusible compound. Accordingly, the action of the above-mentioned development inhibitor material, without staying in a same layer, comes up to the adjacent layers.

Upon this, when the dye produced from the nondiffusible coupler of the present invention is slightly movable as described above, the graininess can be improved by thinly spreading the dye cloud as above-mentioned, and at the same

time the spread of the dye cloud can be controlled to an appropriate extent by the above development control action, and the sharpness can be improved as well. And when the dye produced from the nondiffusible coupler of the invention is completely diffusible, the dye cloud, with further increasing its spread, extends to the adjacent layers, and, when using a mordant as will be described hereinafter, is finally fixed by the mordant, but, during the process, the above dye cloud's spread is appropriately controlled by the development inhibiting action of the above diffusible development inhibitor material, whereby the sharpness as well as the graininess can be improved as described above. The above-mentioned mordant will be described in detail hereinafter.

The movable dye-producible nondiffusible coupler in the present invention has in the coupling position thereof a stabilizing group for rendering the coupler immovable and nondiffusible and in the noncoupling position thereof a control group for controlling the moving degree of the produced dye. When the above coupler couples with the oxidized product of a color developing agent, the stabilizing group is split from the coupler, and the thereby produced dye becomes movable. The moving degree of the movable dye can be controlled by the control group; for example, in one most extreme case, the dye is controlled to become a slightly movable, and in the other most extreme case, it is controlled to become a completely diffusible dye. Such the control group depends on the coupler's mother nucleus, another substituent introduced into the coupler, and the color developing agent used. Even if it is the same substituent, the control group, in the case of one certain coupler, may render the produced dye completely diffusible, and in the case of another, may render the produced dye slightly movable.

The above-described movable dye-reproducible nondiffusible coupler for the present invention has the formula:

Formula (I)

COUP — control group
|
stabilizing group

wherein COUP represents a coupler component for producing a dye, and the stabilizing group is a group that is linked to the above coupler component in the coupling position thereof and can be split from COUP during the coupling reaction between the coupler and the oxidized product of a color developing agent. And the above stabilizing group has such molecular size as enough for rendering the coupler nondiffusible.

The control group is a group that is linked to COUP in the noncoupling position thereof and so controls the dye produced by the coupling reaction of the coupler with the oxidized product of a color developing agent as to become slightly movable as previously described or completely diffusible.

The above COUP represents the coupler component and produces a dye by the reaction thereof with the oxidized product of a color developing agent, so that it may be any known or used coupler component to or by those skilled in the art. For example, the usable yellow dye-producible coupler includes acylanilide-type acetanilides and benzoylacetanilides; the magenta dye-producible coupler includes pyrazolones, pyrazolotriazoles, pyrazolobenzimidazoles and indazolones; and further the cyan dye-producible coupler includes phenols and naphthols. These couplers each is capable of producing the coupler portion COUP.

The aforesaid stabilizing group has sufficient molecular size and form for rendering the coupler nondiffusible. The useful stabilizing group of the kind includes groups having alkyl components and aryl components having not less than 8 carbon atoms, and preferably from 8 to 32 carbon atoms. The stabilizing group may be one that is substituted by such a group as capable of changing the reactivity of the coupler, and may also have a cross-linking group in the coupling position thereof. Those typical cross-linking groups of the kind include, e.g., -O-, -S-, -N=N-, 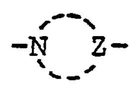 (wherein Z is a group of atoms necessary to form a 5- or 7-member heterocyclic group) and the like. Those preferred stabilizing groups include 8-to-32 carbon atoms-having alkoxy, aryloxy, alkyloxy, arylthio and nitrongen-containing heterocyclic groups.

The foregoing control group, in one extreme case, is a group having molecular size and form suitable for rendering the produced dye slightly movable and, in the other extreme case, is an alkali-soluble group capable of rendering the dye completely diffusible. Those preferred as the above group suitable for rendering the dye slightly movable are alkyl groups having from 1 to 20 carbon atoms and aryl groups having from 6 to 20 carbon atoms. These groups are allowed to be further substituted by a group capable of changing the spectral characteristic and diffusibility of the dye. These control groups are also allowed to have a cross-linking group for linking the same to the coupler's mother nucleus, the cross-lingking group including, e.g., -O-, -S-, -CO-, -COO-, -NR-, -CONR-, -NRCO-, $-SO_2NR-$, $-NRSO_2-$, -NRCONR- (wherein R is a hydrogen atom, an alkyl group or an aryl group), and the like.

The alkali-soluble group rendering the dye completely diffusible, on the other hand, is a group capable of being ionized under a processing condition, and is a group containing, e.g., a hydroxy group, a carboxylic acid group, a sulfonic acid group, or an aminosulfonyl group, and not less than one salt thereof. These groups also are allowed to be ones having a cross-linking group for linking the group to the coupler's mother nucleus, the cross-linking group being typified by, e.g., -O-, -S-, -CO-, -COO-, -NR-, -CONR-, -NRCO-, $-SO_2NR-$, $-NRSO_2-$, -NRCONR- (wherein R is a hydrogen atom, an alkyl

group or aryl group), and the like.

Among the movable dye-producible nondiffusible couplers in the present invention, those preferred yellow couplers have the formula:

Formula (II)

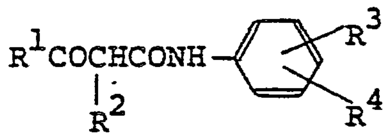

wherein $R^1$ is an aryl group (such as phenyl group) or an alkyl group (such a tertiary alkyl group as t-butyl group); $R^2$ is the foregoing stabilizing group; $R^3$ is the foregoing control group; and $R^4$ represents a hydrogen atom, a halogen atom, an alkyl group or an alkoxy group and a control group.

Those preferred cyan couplers have either one of the formulas:

Formula (III)

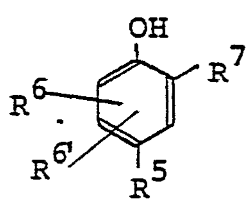

Formula (IV)

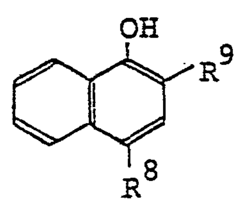

wherein $R^5$ represents a group the same as defined in the $R^2$ of Formula (II); any one of $R^6$, $R^{6'}$ or $R^7$ represents the foregoing control group and the other two of them are allowed to be either the same or different from each other, representing

a hydrogen atom, a halogen atom, an alkyl group, an alkoxy group, an alkylamino group, or an acylamido group; and $R^8$ is a group as defined in $R^5$; and $R^9$ represents a control group.

Those preferred magenta couplers have either one of the formulas:

Formula (V)

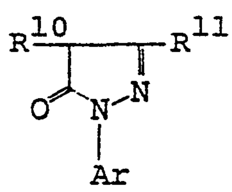

Formula (VI)

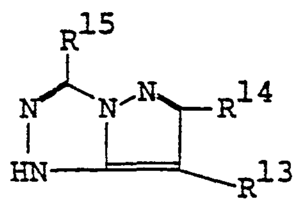

wherein $R^{10}$ represents a group as defined in the $R^5$ of Formula (III); $R^{11}$ represents a control group; Ar is a phenyl group which is allowed to have at least one substituent selected from the class consisting of a hydrogen atom, alkyl groups, alkoxy groups and amino groups, the phenyl group being allowed to have the foregoing control group; $R^{13}$ is a group as defined in $R^{10}$; and either one of $R^{14}$ and $R^{15}$ is a control group and the other is a hydrogen atom, an alkyl group, an alkoxy group, an aryl group, an amino group, or an acylamido group.

Except those particularly mentioned, the above alkyl, alkoxy, and alkylamino groups each has from 1 to 8 carbon atoms, the aryl groups each has from 6 to 10 carbon atoms, and the amino groups include primary, secondary, and tertiary amino groups. These groups and control groups are allowed to be further substituted by a halogen atom, a hydroxy group, a carboxy group, an amino group, an amido group, a carbamoyl group,

a sulfoamoyl group, a sulfonamido group, an alkyl group, an alkoxy group, an aryl group, or the like.

The following are typical examples of the movable dye-producible nondiffusible couplers in the present invention, but the present invention is not limited thereto.

( C − 1 )

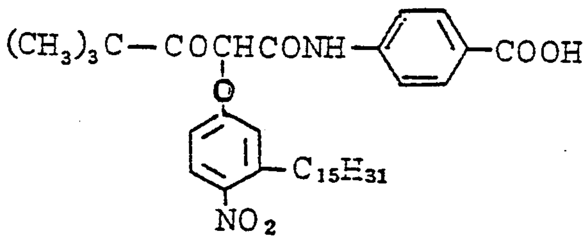

( C − 2 )

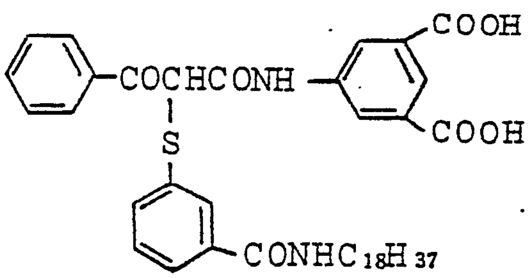

( C − 3 )

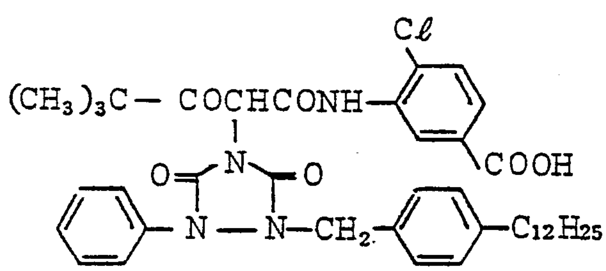

( C − 4 )

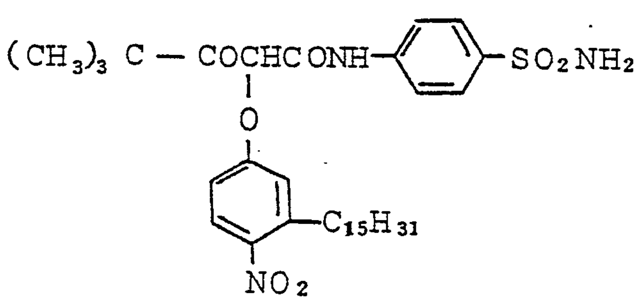

( C − 5 )

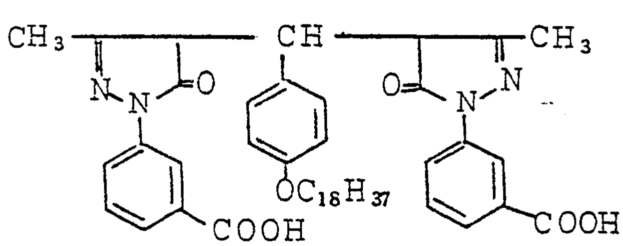

( C — 6 )

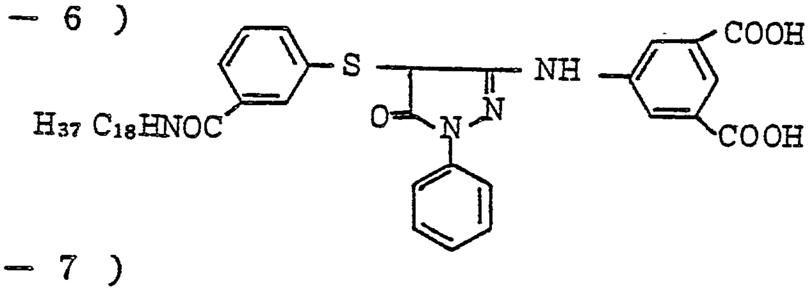

( C — 7 )

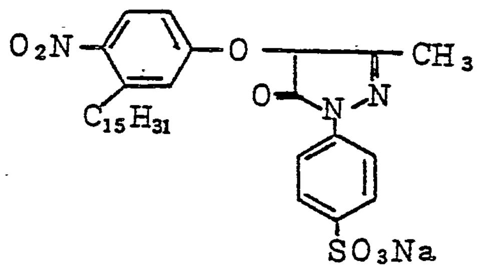

( C — 8 )

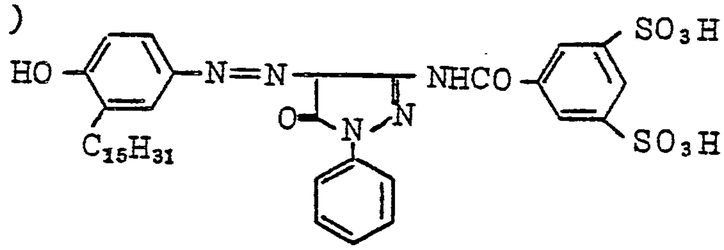

( C — 9 )

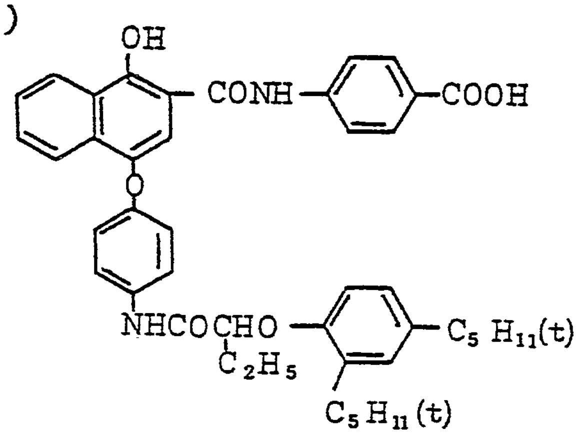

( C — 10 )

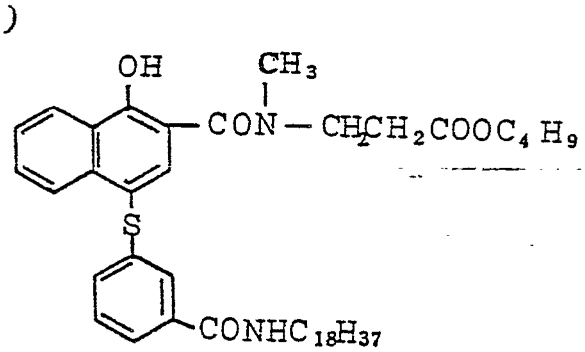

( C - 11 )

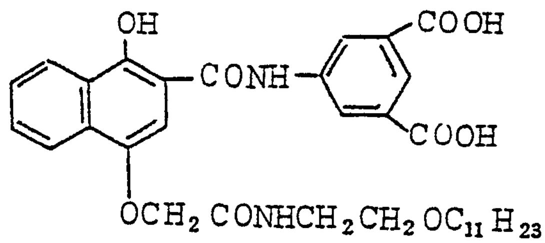

( C - 12 )

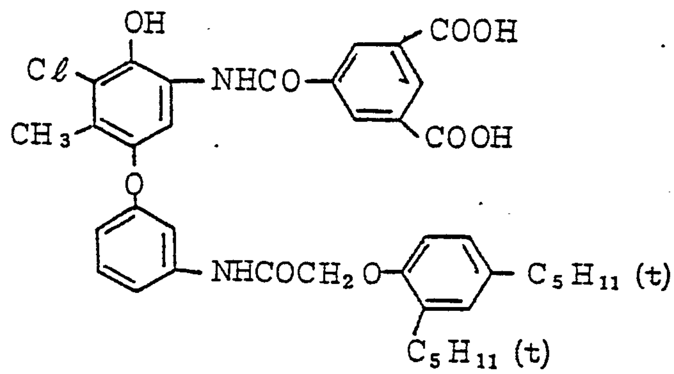

( C - 13 )

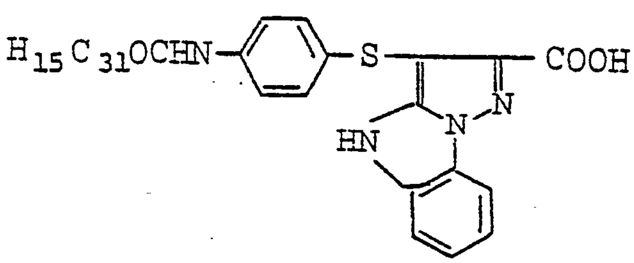

( C - 14 )

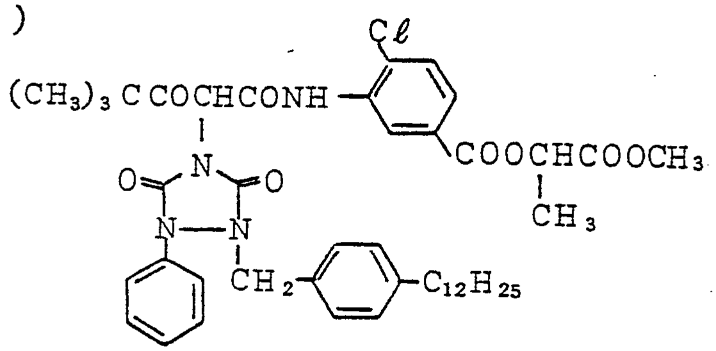

( C − 15 )

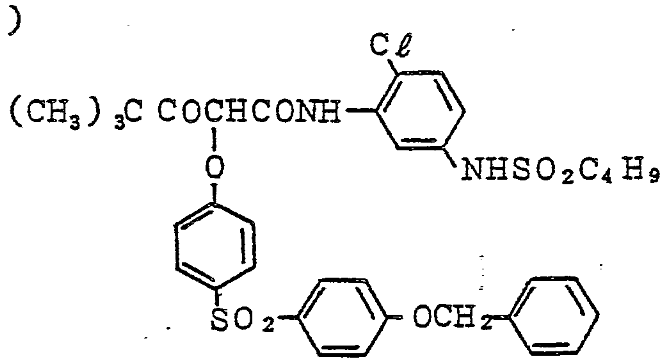

( C − 16 )

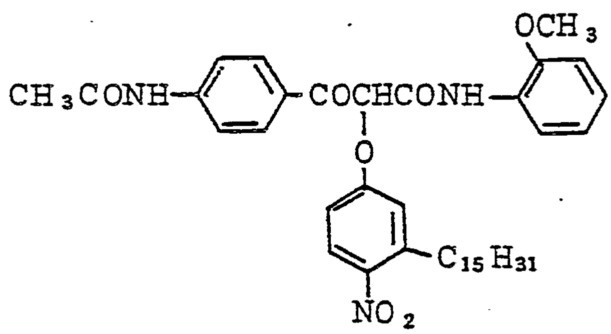

( C − 17 )

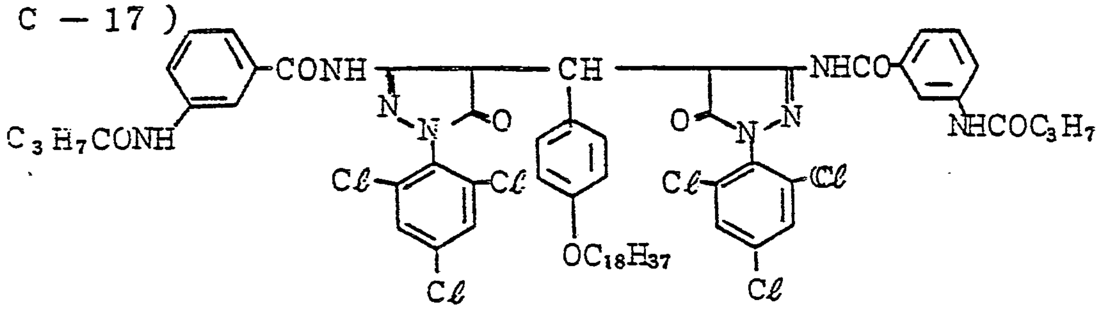

( C − 18 )

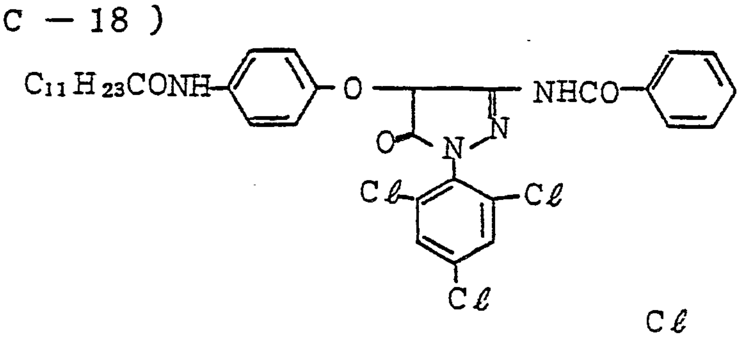

( C − 19 )

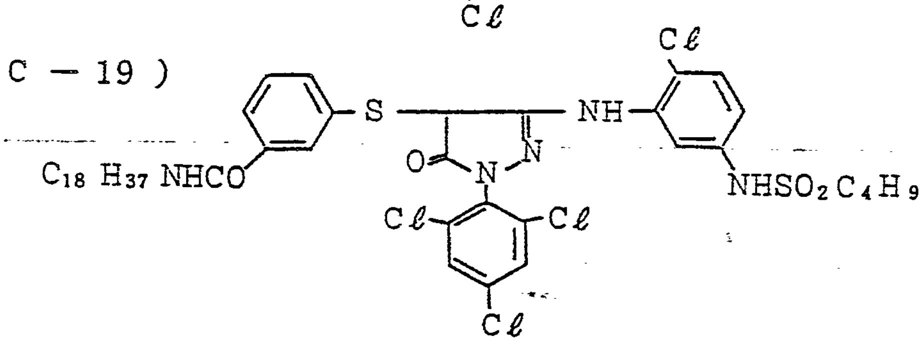

( C — 20 )

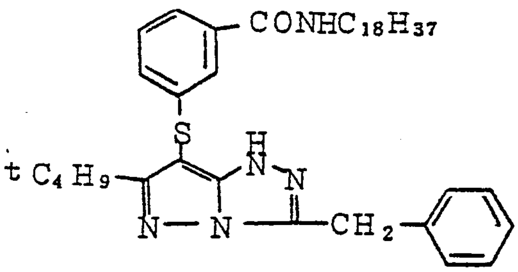

( C — 21 )

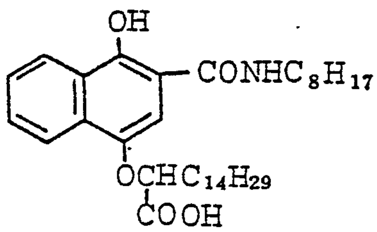

( C — 22 )

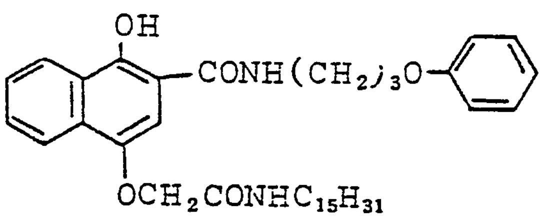

( C — 23 )

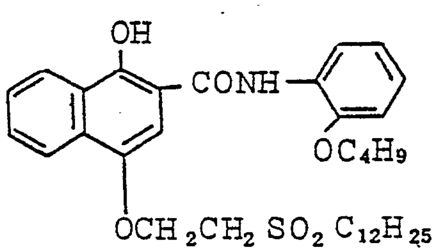

( C — 24 )

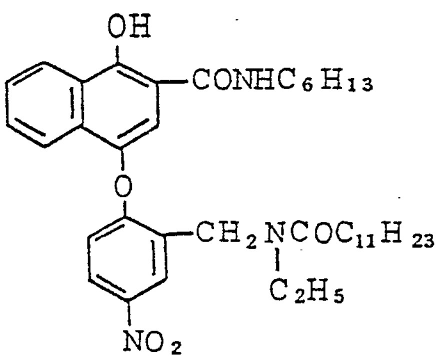

( C — 25 )

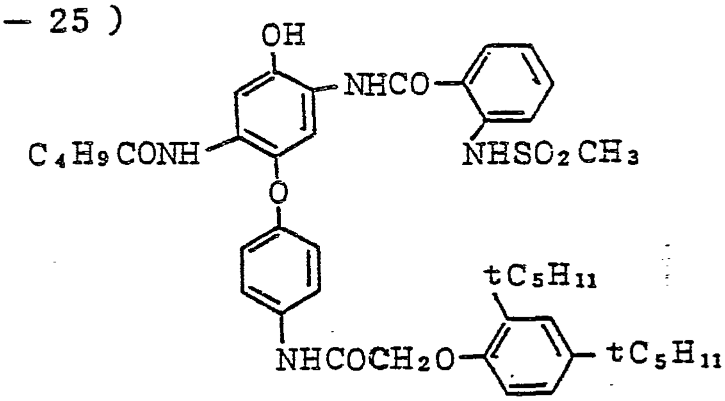

( C — 26 )

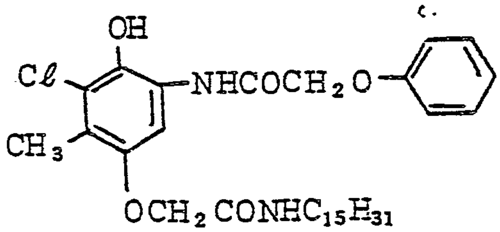

( C — 27 )

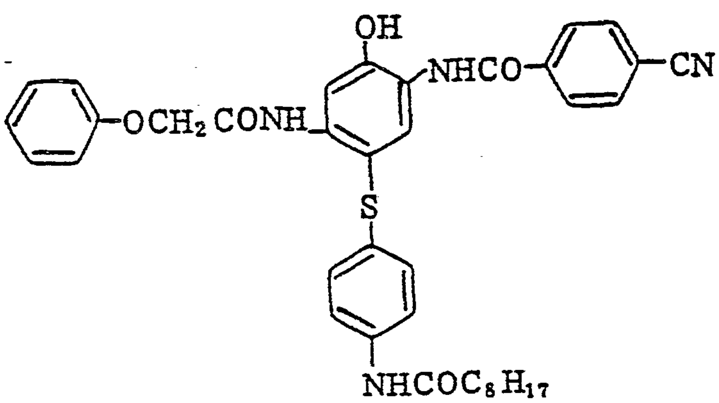

Typical synthesis examples of some of the above compounds will be subsequently described below. Those compounds not described in the following synthesis examples can also be easily synthesized in similar manners to the following examples.

## SYNTHESIS EXAMPLE 1

Synthesis of Exemplified Compound C-3:

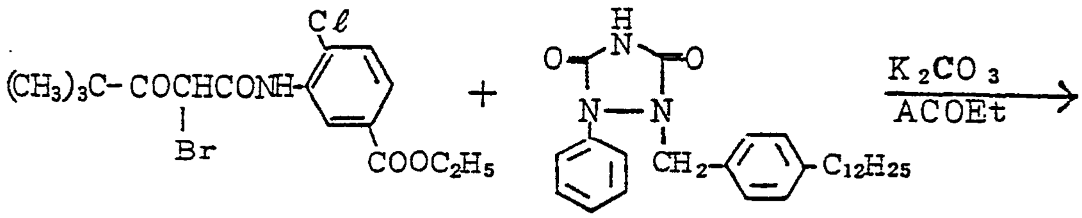


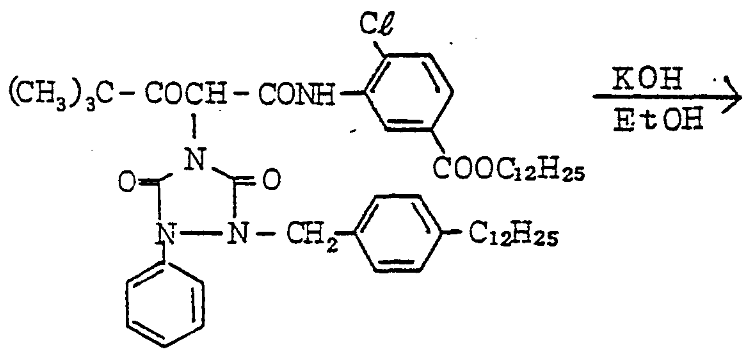


Exemplified Compound C-3

(Compound 1)

(Synthesis of Compound 1)

Seven point four grams (0.018 mole) of α-pivaloyl-α-bromo-2-chloro-5-ethoxycarbonyl-acetanilide and 8.0g (0.018 mole) of 3-phenyl -4-(4-dodecyl-benzyl)-urazole are dissolved into 100ml of ethyl acetate, and to the solution are added 1.3g (0.009 mole) of anhydrous potassium carbonate, and the mixture is refluxed by heating for three hours. To the reaction product are added 200ml of water to extract the ethyl acetate stratum, which is then concentrated to thereby obtain an yellow viscous substance as Compound 1.

(Synthesis of Exemplified Compound C-3)

The thus obtained Compound 1 is dissolved into 50ml of ethyl alcohol, and to the solution is added a solution of 5.0g

of potassium hydroxide dissolved into 10ml of water to effect a reaction for two hours. The resulting reaction product is added to an iced water containing 10ml of concentrated hydrochloric acid with stirring, whereby a white solid product is deposited, which is filtrated, washed, and then dried, and after that, is recrystallized with use of ethyl actate-n-hexane, whereby 10.3g of Exemplified Compound C-3 are obtained.

SYNTHESIS EXAMPLE 2

Synthesis of Exemplified Compound C-5:

Ten point six grams (0.024 mole) of 1-(3-carboxyphenyl)-3-methyl pyrazolone and 9.1g (0.012 mole) of 4-octadecyloxybenzaldehyde are dissolved into 200ml of ethyl alcohol, and to the solution are added three drops of triethylamine, and the reaction of the mixture takes place for 5 hours. After concentration, the resulting solid substance is washed with ethyl acetate to thereby obtain 14.6g of Exemplified Compound C-5.

SYNTHESIS EXAMPLE 3

Synthesis of Exemplified Compound C-9:

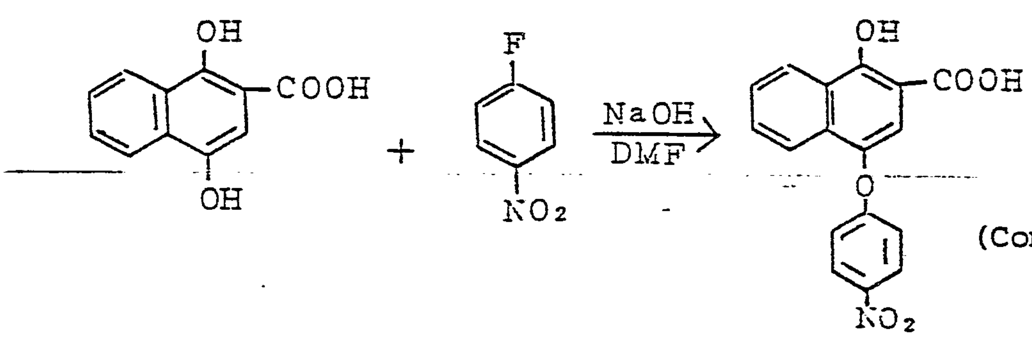


(Compound 2)

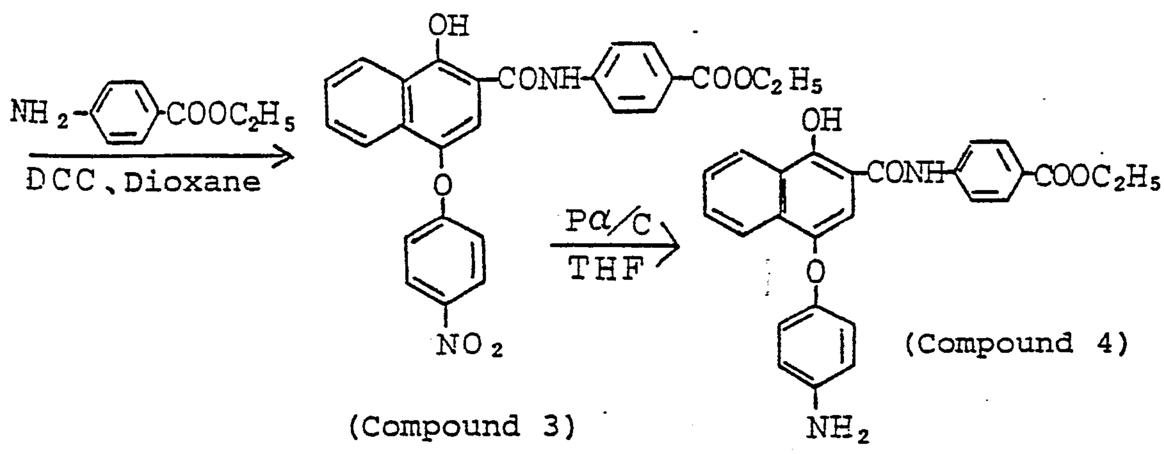


(Compound 3)

(Compound 4)

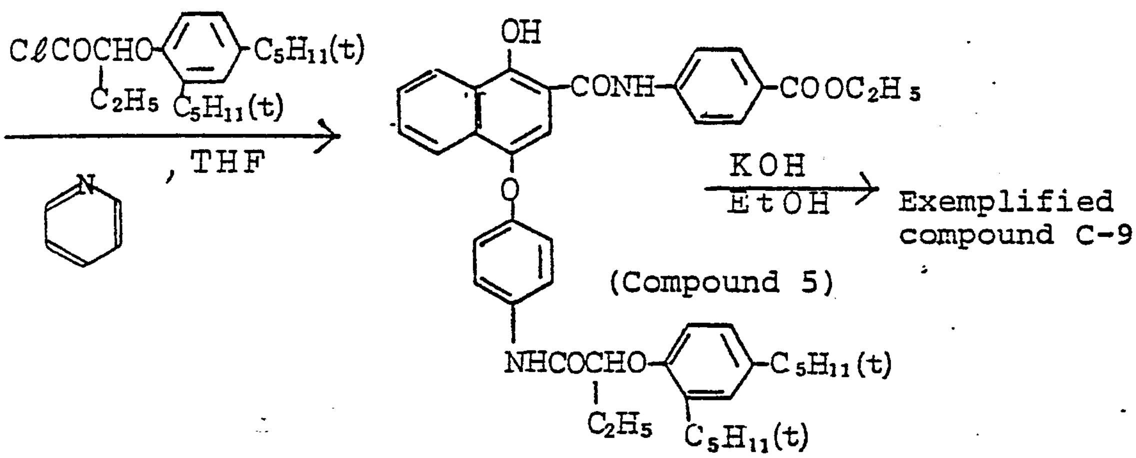


(Compound 5)

(Synthesis of Compound 2)

Twenty point four grams (0.1 mole) of 1,4-dihydroxy-2-naphthoic acid and 14.1g (0.1 mole) of p-nitrofluorobenzene are dissolved into 300ml of dimethylformamide, and to the solution, with conducting a nitrogen gas thereinto, is added a solution of 8.5g (0.2 mole) of sodium hydroxide dissolved into 20 ml of water, and the reaction of the mixture takes place for an hour. The resulting reaction product is added to iced water containing 20ml of concentrated hydrochloric acid with stirring, thereby producing a thick syrup-like solid,

which is then heated over a water bath with stirring to thereby become solidified. The resulting crystals are filtrated, washed with water and then with acetonitrile, and then dried, whereby 23.2g of a light yellow solid substance as Compound 2 were obtained.

(Synthesis of Compound 3)

Twenty-one point of eight grams (0.067 mole) of the above-obtained Compound 2 and 11.1g (0.067 mole) of ethyl p-aminobenzoate are dissolved into 200ml of dioxane, and to the mixture are added 13.8g (0.067 mole) of N,N'-dicyclohexylcarbodiimide, and the reaction of the mixture takes place for two hours. The deposited urea is filtered off and then washed three times with 200ml of heated dioxane. The filtrate is concentrated, and the resulting solid substance is washed with heated ethyl acetate, whereby 21g of an yellowish green solid as Compound 3 are obtained.

(Synthesis of Compound 4)

Twenty-one grams of the above-obtained Compound 3 are dissolved into 450 ml of tetrahydrofuran, and to the solution is made catalytic hydrogenation with use of 4g of a 5% palladium carbon catalyst over a period of 10 hours. The catalyst is removed and the liquid is concentrated, and after that the obtained solid is washed with ethyl alcohol, whereby 9.2g of

Compound 4 are obtained.

(Synthesis of Compound 5)

Nine point two grams (0.021 mole) of the above-obtained Compound 4 and 7.1g (0.021 mole) of α-(2,4-di-t-pentylphenoxy)-butyroyl chloride are dissolved into 100ml of tetrahydrofuran, and to the solution are added 1.7g (0.021 mole) of pyridine, and the reaction of the mixture takes place for two hours. The produced pyridine hydrochloride is filtered off, and the filtrate is concentrated to thereby produce a redish-brown viscous product, which is then subjected to a silica gel column treatment with use of a mixture solvent of chloroform with n-hexane in the proportion of 1 : 1, whereby 10g of a light yellow thick syrup-like product as Compound 5.

(Synthesis of Exemplified Compound C-9)

Seven grams of the above-obtained Compound 5 are dissolved into 50ml of ethyl alcohol, and to the solution is added a solution of 6.0g of potassium hydroxide dissolved into 10ml of water, and the reaction of the mixture takes place for three hours. The reaction product is then poured, with stirring, into an iced water containing 10ml of concentrated hydrochloric acid, whereby white crystals are produced. The crystals are filtered, washed with water and then with acetonitrile, and further recrystallized from an acetonitrile-ethyl acetate mixture liquid, whereby 4.7g of Exemplified Compound C-9 are obtained.

The structure of the above-obtained objective compound was confirmed by mass spectrometry.

The movable dye-producible nondiffusible coupler used in the present invention may be either one that produces a slightly movable dye or one that produces a completely diffusible dye, or both may be used together. In the case of using the completely diffusible dye-producible nondiffusible coupler, a mordant layer to be paired with the coupler is used, which mordant layer may be provided immediately on an emulsion containing this coupler or may also be disposed through such an interlayer as an inert gelain layer, or allowed to be the topmost layer through some further emulsion layers. As another method, the above mordant may be incorporated together with the coupler into the same layer.

The DIR compound contained in the foregoing highest-speed emulsion layer and used in the present invention is a compound capable of releasing a development inhibitor material by the reaction thereof with the oxidized product of a color developing agent.

As typical ones of such the DIR compound, there are those so-called DIR couplers into the active site of which is introduced a group capable of forming a compound having development inhibitability when split from the active site. Those DIR couplers of the kind are described in, e.g., British Patent No. 953,454,

U.S. Patent Nos. 3,227,554, 4,095,984 and 4,149,886.

The above-mentioned DIR couplers have the nature that the coupler's mother nucleus forms a dye during the coupling reaction thereof with the oxidizied product of a color developing agent, and concurrently releases a development inhibitor material. The present invention also includes those compounds which, during the coupling reaction thereof with the oxidized product of a color developing agent, releases a development inhibitor material but do not form any dye, as described in U.S. Patent Nos. 3,652,345, 3,928,041, 3,958,993, 3,961,959 and 4,052,213, and Japanese Patent O.P.I. Publication Nos. 110529/1978, 13333/1979 and 161237/1980.

Further, the present invention includes the so-called timing DIR compounds wherein, during the reaction thereof with the oxidized product of a color developing agent, the mother nucleus forms a dye or a colorless compound, while the split--off timing group, by the intramolecular nucleophilic substitution reaction or split-off reaction, releases a development inhibitor material, as described in Japanese Patent O.P.I. Publication Nos. 145135/1979 and 114946/1981 and Japanese Patent Application No. 39766/1981.

Furthermore, the invention also includes those timing DIR compounds as described in Japanese Patent Application Nos. 44831/1982 and 45807/1982, wherein, during the reaction thereof with the oxidized product of a color developing agent, the

completely diffusible dye-producible coupler's mother nucleus has the foregoing timing group linked thereto.

According to the present invention, those more preferred DIR compounds are represented by the following Formula (VII) or Formula (VIII):

Formula (VII) COUP - inhibitor

wherein COUP represents a coupler component capable of coupling with the oxidized product of a color developing agent, the component including such dye-formable couplers as, e.g., open-chain ketomethylene compounds of acylacetanilides, pyrazolones, pyrazolotriazoles, pyrazolobenzimidazoles, indazolones, phenols, naphthols, and the like, and such coupling components substantially not forming any dye as actophenones, indanones, oxazolones, and the like.

The inhibitor in the above formula is a compound that inhibits the development of silver halides, and those preferred compounds of the kind include such heterocyclic compounds and heterocyclic mercapto compounds as benzotriazole, 3-octylthio-1,2,4-triazole, and the like.

The above heterocyclic group includes tetrazolyl groups, thiadiazolyl groups, oxadiazolyl groups, thiazolyl groups, oxazolyl groups, imidazolyl groups, triazolyl groups, and the like; and to be more concrete, 1-phenyl-tetrazolyl group, 1-ethyl-tetrazolyl group, 1-(4-hydroxyphenyl)tetrazolyl group, 1,3,4-thiazolyl group, 5-methyl-1,3,4-oxadiazolyl group, benzothiazolyl group, benzoxazolyl group, benzimidazolyl group, 4H-1,2,4-triazolyl group, and the like.

Formula (VIII)

COUP - TIME - inhibitor

wherein inhibitor is as defined in Formula (VII); COUP, in addition to being as defined in Formula (VII), also includes a completely diffusible dye-producible coupler component; and TIME includes those having the following Formulas (IX), (X) and (XI), but is not limited thereto:

Formula (IX)

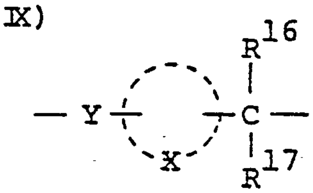

wherein X is a group of atoms necessary to complete a benzene or naphthalene ring; Y is -O-, -S-, or $\overset{R^{18}}{\overset{|}{-N-}}$ (wherein $R^{18}$ is a hydrogen atom, an alkyl group or an aryl group), any of which is linked to the ring in the coupling position thereof; and $R^{16}$ and $R^{17}$ each is as defined in the $R^{18}$, but the group of $-\underset{R^{17}}{\overset{R^{16}}{C}}-$ is substituted in the ortho or para position to the Y and bonded with the hetero atom contained in the inhibitor.

Formula (X)

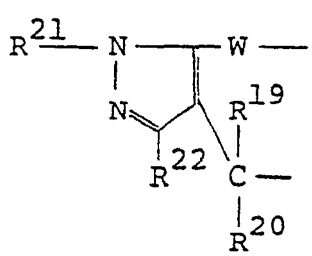

wherein W is a group as defined in the Y of Formula (IX); $R^{19}$ and $R^{20}$ are as defined in the $R^{16}$ and $R^{17}$, respectively, of Formula (IX); $R^{21}$ is a hydrogen atom, an alkyl group, an aryl group, an acyl group, a sulfone group, an alkoxycarbonyl group or a heterocyclic residue; and $R^{22}$ is a hydrogen atom, an alkyl group, an aryl group, heterocyclic residue, an alkoxy group, an amino group, an acylamido group, a sulfonamido group, carboxy group, an alkoxycarbonyl group, a carbamoyl group, or a cyano group. And the timing group is linked by W to COUP in the coupling position thereof and bonded by the $-\overset{R^{19}}{\underset{R^{20}}{C}}-$ to the hetero atom of the inhibitor.

The following is an exemplified Formula (XI) representing those timing groups releasing the inhibitor by the intramolecular nucleophilic substitution reaction:

Formula (XI)

$$\begin{array}{l} Nu \\ | \\ V-E- \end{array}$$

wherein Nu is an electron-rich nucleophilic group having an oxygen, sulfur or nitrogen atom and is linked to COUP in the coupling position thereof; E is an electron-poor electrophilic group having a carbonyl, thiacarbonyl, phosphonyl or thiophosphinyl group and is bonded to the hetero atom of the inhibitor; and V is a linkage group which establishes the steric relation of Nu with E, and which, after Nu is released from COUP, is subjected to the intramolecular nucleophilic

substitution reaction accompanied by the formation of 3- to 7-member cyclic ring, and is thereby capable of releasing an inhibitor.

The following are typical examples of the DIR compound of the present invention, but the present invention is not limited thereto.

Exemplified Compound:

( D − 1 )

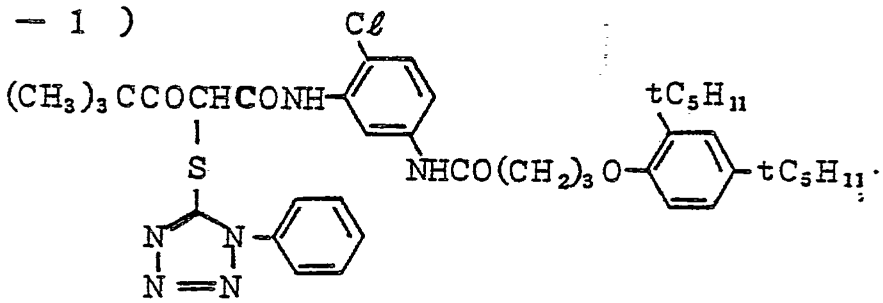

( D − 2 )

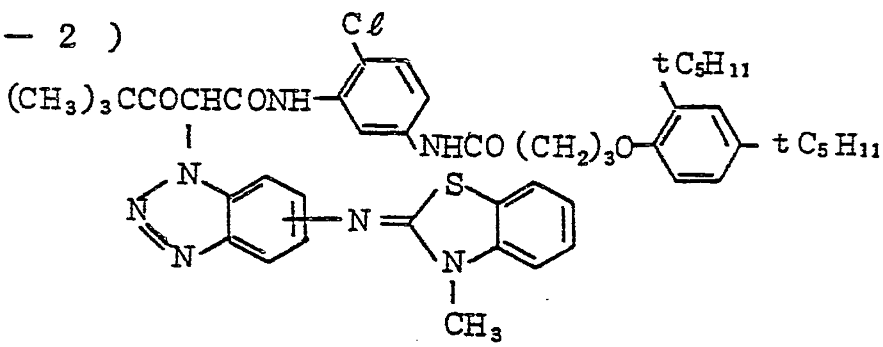

( D − 3 )

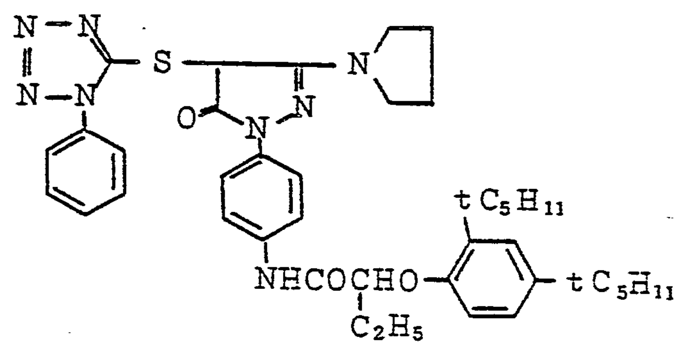

( D − 4 )

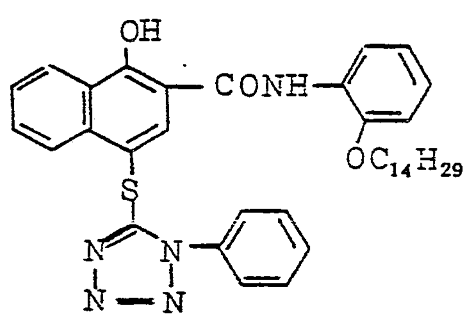

( D − 5 )

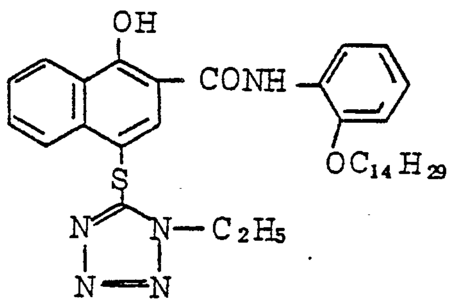

( D − 6 )

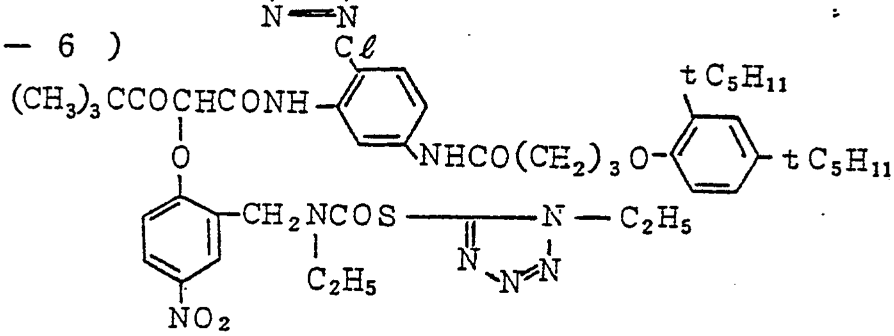

( D − 7 )

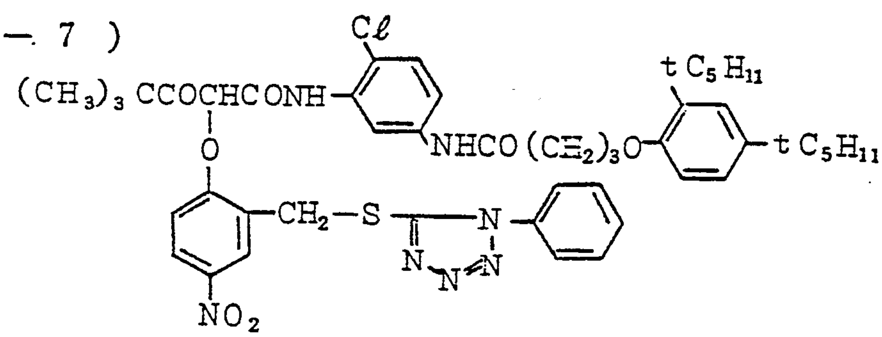

( D − 8 )

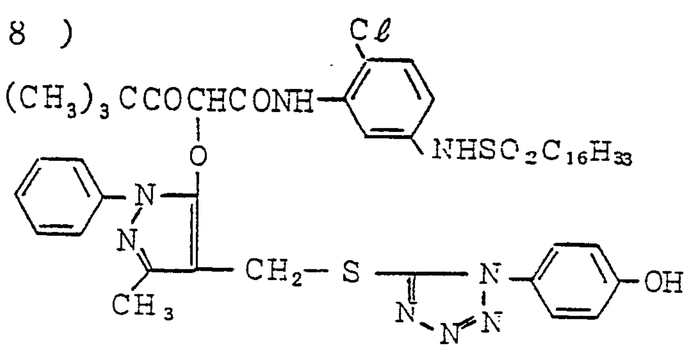

( D - 9 )

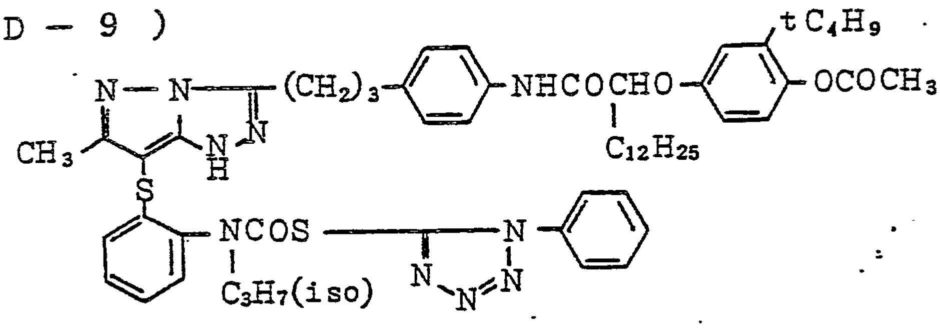

( D - 10 )

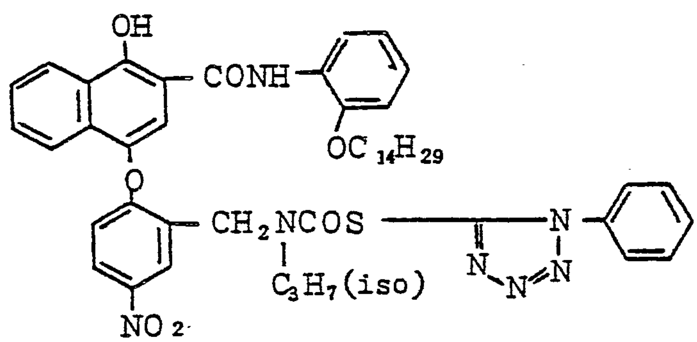

( D - 11 )

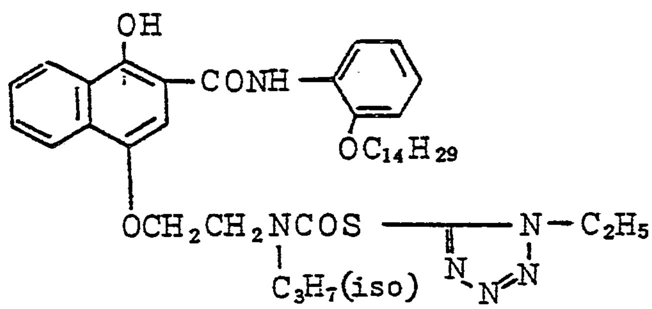

( D - 12 )

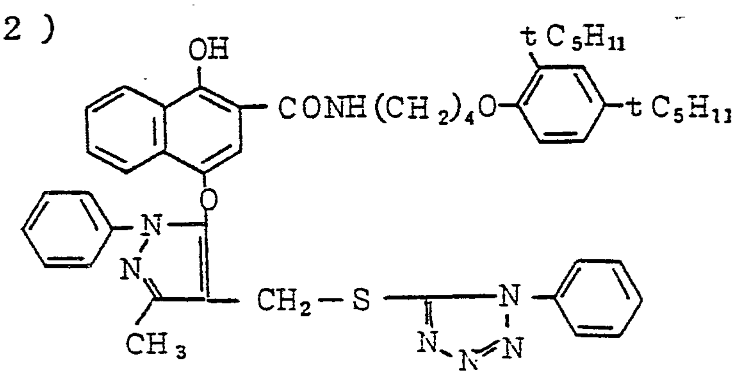

( D − 13 )

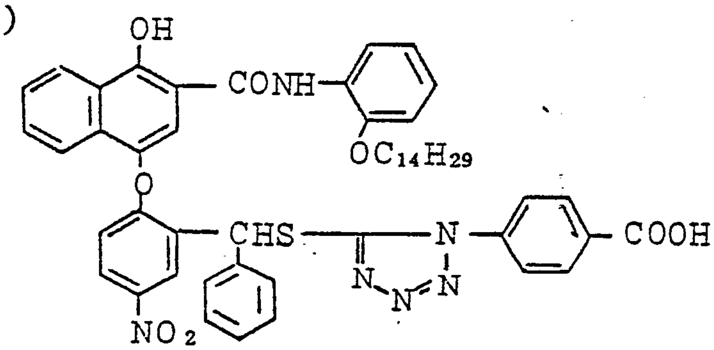

( D − 14 )

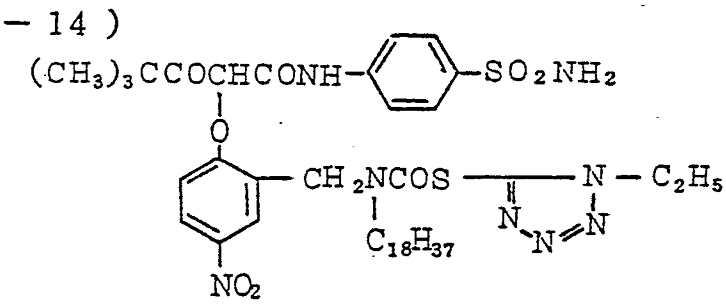

( D − 15 )

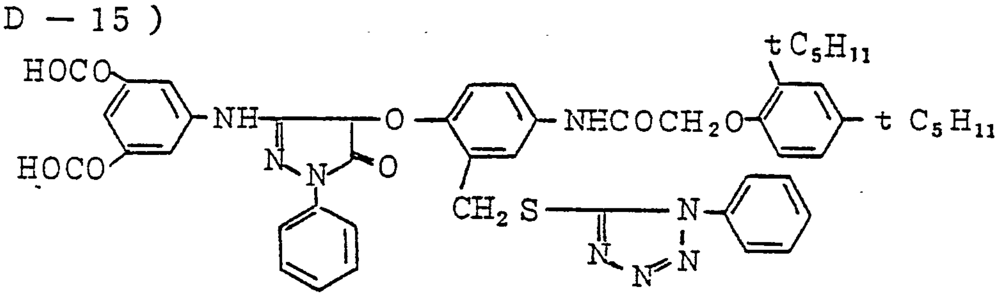

( D − 16 )

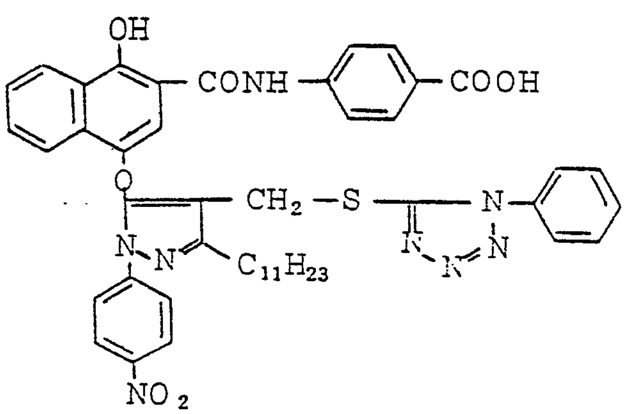

( D − 17 )

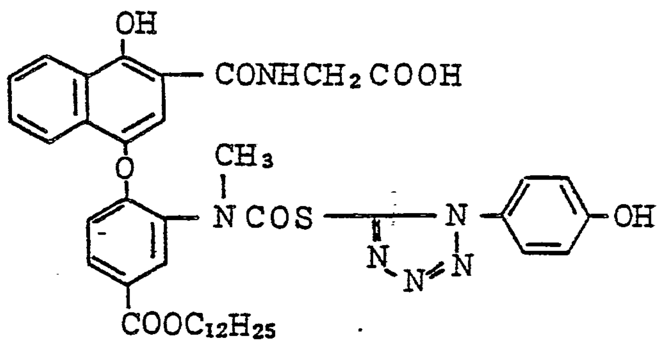

( D − 18 )

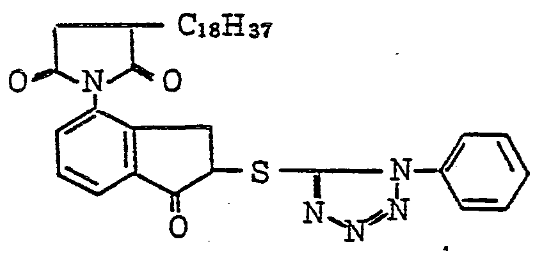

( D − 19 )

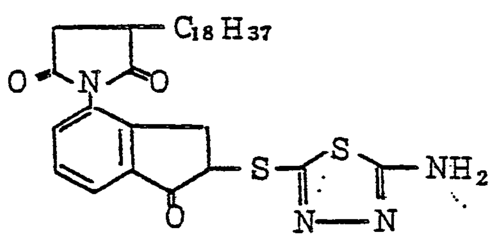

( D − 20 )

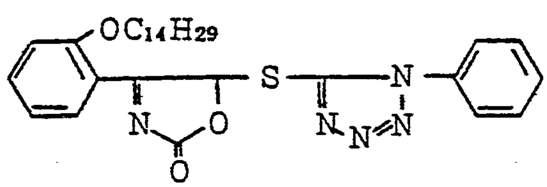

( D − 21 )

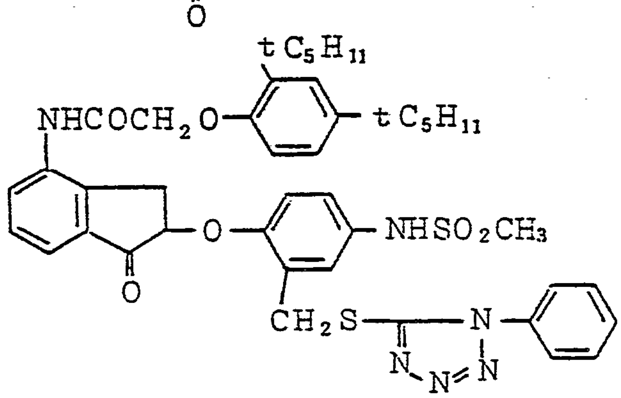

These DIR compounds can be synthesized in such manner as described in U.S. Patent Nos.3,227,554, 3,615,506, 3,632,345, 3,928,041, 3,933,500, 3,938,996, 3,958,993, 3,961,959, 4,046,-574, 4,052,213, 4,063,950, 4,095,984 and 4,149,886, Japanese Patent O.P.I. Publication Nos.81144/1975, 81145/1975, 13239/-1976, 64927/1976, 104825/1976, 105819/1976, 65433/1977, 82423/-1977, 117627/1977, 130327/1977, 154632/1977, 7232/1978, 9116/-1978, 29717/1978, 70821/1978, 103472/1978, 110529/1978, 135333/-1978, 143223/1978, 13333/1979, 49138/1979, 114241/1979, 145135/-1979, 161237/1980 and 114946/1981, and Japanese Patent Application Nos.39766/1981, 44831/1982 and 45807/1982, and the like.

The nondiffusible dye-producible nondiffusible coupler used for both high-speed emulsion layer and low-speed emulsion layer in the present invention may be any of known dye-producible couplers capable of producing appropriate color tone-having nondiffusible dyes. Such the coupler is incorporated at least into a lower-speed silver halide emulsion layer, but allowed to be present in the highest-speed silver halide emulsion layer containing a movable dye-producible nondiffusible coupler.

In the present invention, the quantity of the foregoing movable dye-producible nondiffusible coupler to be incorporated into the highest-speed silver halide emulsion layer is within the range of from $2x10^{-3}$moles to $2x10^{-1}$moles per mole of silver, and the preferred quantity range of the DIR compound to be contained in the same layer is generally from $10^{-3}$ mole to $10^{-1}$mole per mole of silver.

When using the nondiffusible dye-producible nondiffusible coupler in the above emulsion layer, the using quantity may be selected in the range of from 1/50 to 10 times the quantity of the above movable dye-producible nondiffusible coupler.

In the present invention, the mordant used in a pair with the completely diffusible dye-producible nondiffusible coupler may be any mordant capable of fixing the dye produced as a result of the coupling reaction. Those preferred mordants include such basic polymer mordants as described in U.S. Patent No.3,958,995, Japanese Patent O.P.I. Publication Nos.74430/1979 and 22766/1980, such polymers of aminoguanidine derivatives of vinyl-methyl ketones as described in U.S. Patent No.2,882,-156, and such basic polymer mordants as described in U.S. Patent Nos.3,625,394, 2,709,590 and 3,393,033, and the like. Other useful mordants are described in U.S. Patent No.3,559,095 and on pages 30 - 32 of Research Disclosure Dec. 1976. These mordants may be either added to a silver halide emulsion layer or formed as an independent layer.

A gelatin inert layer and a silver halide emulsion layer may be present between the above-mentioned completely diffusible dye-producible nondiffusible coupler layer and the mordant layer, but the larger the space between the coupler and mordant layers, the more becomes the sharpness of the resulting dye image deteriorated. In order to minimize such deterioration of the sharpness, the mordant may be mixed into the coupler-containing layer as described above. The using quantity of the foregoing mordant may be from 0.1 to $5g/m^2$, and preferably from 0.3 to $1.5g/m^2$.

For the silver halide photographic light-sensitive material of the present invention there may be used colored couplers as masking couplers. As a colored magenta coupler as the masking coupler, a compound produced by the substitution of an arylazo group at the active site of a colorless magenta coupler is generally used, which includes those compounds as described in, e.g., U.S. Patent Nos.2,801,171, 2,983,608, 3,005,712 and 3,684,514, British Patent No.937,621, and Japanese Patent O.P.I. Publication Nos.123625/1974 and 131448/1974, and the like.

Further, there may also be used colored magenta couplers of the type that by the reaction thereof with the oxidized product of a color developing agent the produced dye runs out into a processing bath, as described in U.S. Patent No.3,419,-391.

As a colored cyan coupler as the masking coupler there may be used a compound produced by the substitution of an arylazo group at the active site of a colorless cyan coupler is used, which includes those compounds as described in, e.g., U.S. Patent Nos.2,521,908 and 3,034,892, British Patent No.

1,255,111, and Japanese Patent O.P.I. Publication No.22028/-1973.

Further, there may also be used those colored cyan couplers of the type that by the reaction thereof with the oxidized product of a color developing agent the formed dye runs out into a processing bath, as described in U.S. Patent No. 3,476,563, and Japanese Patent O.P.I. Publication Nos.10135/-1975 and 123341/1975.

In order to improve the photographic characteristics, the photographic light-sensitive material may also contain a colorless dye-formable coupler, the so-called competing coupler.

The silver halide photographic emulsion for use in the present invention comprises a hydrophilic macromolecular material such as gelatin into which is dispersed in the colloidal particle-form silver chloride, silver bromide, silver chlorobromide, silver chloroiodide, silver iodobromide, or silver chloroiodobromide, which may be prepared in various manners.

The above silver halide photographic emulsion can be sensitized by chemical sensitizers of the prior art, which include noble-metallic sensitizers, sulfur sensitizers, selenium sensitizers, and reduction sensitizers, which may be used singly or in combination. Further, the silver halide photographic emulsion of the present invention may, if necessary, be spectrally sensitized by use of sensitizing dyes of the prior art.

It may be desirable for displaying sufficiently the effect of the present invention to use in the light-sensitive silver halide emulsion layer or the hydrophilic colloidal layer adjacent thereto by incorporating in combination a reducing agent or oxidation inhibitor including, e.g., sulfites (sodium sulfite, potassium sulfite, etc.), hydrogensulfites (sodium hydrogensulfite, potassium hydrogensulfite, etc.), hydroxylamines (hydroxylamine, N-methyl-hydroxylamine, etc.), sulfinates (sodium phenyl-sulfinate, etc.), hydrazines (N,N'-dimethyl-hydrazine, etc.), reductones (ascorbic acid, etc.), aromatic hydrocarbons having at least one hydroxyl group (p-aminophenol, gallic acid, catechol, pyrogallol, resorcinol, 2,3-dihydroxynaphthalene, etc.), and the like.

Further, in order to improve the light resistance of the magenta dye image formed from the magenta coupler used in the present invention, there may be incorporated into the emulsion layer or the adjacent layers thereto p-alkoxyphenols or phenolic compounds.

The construction of the silver halide color photographic light-sensitive material of the present invention may be on the basis of the subtractive color process. As a rule, the fundamental construction of the material is comprised principally of three layers: a blue-sensitive layer containing an yellow coupler for the formation of an yellow dye, a green-sensitive layer containing a magenta coupler for the formation of a magenta dye, and a red-sensitive layer containing a cyan

coupler for the formation of a cyan dye. Further, at least one of these three layers or all the layers each is desirable to be composed of two or three superposed layers to thereby improve such photographic characteristics as the color-forming characteristic, color reproductions, graininess of the color-formed dyes, and the like.

In addition to these fundamental emulsion layers, there may be appropriately used a protective layer as the topmost layer, interlayers and filter layers between the emulsion layers, subbing layer as the bottom layer, and further an antihalation layer to thereby enable to improve the protection, prevention of color stain, graininess, color reproductions, layer adhesiveness of the light-sensitive material.

These emulsion layers and other layers are coated by any of known methods on an appropriate support material such as laminated paper, cellulose acetate, polystyrene, or the like.

In order to prevent possible deterioration of the speed or occurrence of fog during the manufacture, storage, or processing of the color light-sensitive material, to the silver halide emulsion may be added various compounds including such a heterocyclic compound as, e.g., 1-phenyl-5-mercaptotetrazole, 3-methyl-benzothiazole, 4-hydroxy-6-methyl-1,3,3a,7-tetrazaindene, etc., a mercapto compound, a metallic salt, and the like. And the emulsion may be subjected to a hardening treatment in accordance with a usual manner.

To the above silver halide emulsion may be added a single surfactant or a mixture of surfactants. As the surfactant, there may be used various active agents as coating aid, emulsifying agent, improving agent for the permeability of processing liquids into the emulsion, defoaming agent, antistatic agent, anti-adhesion agent, or for improving the photographic characteristics or controlling the physical property of the emulsion.

In the silver halide photographic light-sensitive material of the present invention, in order to prevent possible occurrence of unnecessary fog or stain due to the air oxidation of an aromatic primary amino developing agent or to prevent possible diffusion of the oxidized product of the color developing agent into the adjacent layers during processing, it is advantageous to use in the silver halide emulsion layers or interlayers those alkyl-substituted hydroquinone compounds as desclosed in U.S. Patent Nos.2,728,659, 2,732,300 and 3,700,453, and Japanese Patent O.P.I. Publication No.15438/-1975 and Japanese Patent Application No.2551/1979.

To incorporate into the silver halide photographic light-sensitive material the compounds to be contained in the high-speed emulsion layer of the present invention, the compounds may be incorporated in various manners into the coating liquids of the component layers to contain the same, and to the incorporation may be applied various techniques that have conventionally been used for couplers; for example, the incorporation is made by dissolution into a high-boiling solvent as described in U.S. Patent No.2,322,027; a coupler and a high-boiling solvent are separately dispersed in the finely particulate form, and then mixed to be incorporated as described in U.S. Patent No.2,322,027; or in the dispersing method, the use of a low-boiling solvent may be advantageous. In this instance, the compounds of the present invention can be mixed with the coupler to be dispersed or can also be dispersed separately from the dispersion of the coupler and then used together. In the case of using a low-boiling solvent, it is possible to remove the low-boiling solvent from the dispersed liquid by such a method as described in U.S. Patent No.2,801,-171 or in Japanese Patent Examined Publication No.8099/1974.

Those particularly preferred among the applicable solvents to the invention include, as high-boiling solvents, dibutyl phthalate, dioctyl phthalate, diisodecyl phthalate, triphenyl phosphate, tricresyl phosphate, diethyl laurylamide, dibutyl laurylamide, benzyl phthalate, monophenyl-p-t-butylphenyl phosphate, phenoxyethanol, diethylene-glycol-monophenyl ether, di-methoxyethyl phthalate, hexamethylphosphoramide, and further those high-boiling organic solvents not miscible with water as described in U.S. Patent No.3,779,765, Japanese Patent O.P.I. Publication No.90523/1974, and Japanese Patent Examined Publication No.29060, and as low-boiling solvents,

for example, methyl-isobutyl ketone, β-ethoxyethyl acetate, methoxytriglycol acetate, acetone, methyl acetone, methanol, ethanol, acetonitrile, dioxane, dimethylformaide, dimethylsulfoxide, ethyl acetate, butyl acetate, isopropyl acetate, butanol, chloroform, cyclohexane, cyclohexanol, fluorinated alcohol, and the like, and any of these low-boiling solvents may be used in place of or by mixing with any of the foregoing high-boiling solvents. Further, these solvents may be used singly or in combination of not less than two.

In addition, as another method, in the case of a watersoluble group-having coupler and of the compound of the present invention, the Fischer-type method, i.e., a method wherein they are dissolved into an alkaline liquid thereby to be used can be applied, or alternatively, a method wherein either one of the coupler and the compound of the invention may be incorporated by the dispersion method and the other by the Fischer-type method into the same layer.

A color developer for use in developing the silver halide photographic light-sensitive material of the present invention is an aqueous alkaline solution containing a color developing agent, whose pH is not less than 8, and preferably from 9 to 12. An aromatic primary amino developing agent as the developing agent means a compound or a precursor forming such the compound having a primary amino group and being capable of developing an exposed silver halide to light.

The above-mentioned developing agent is typified by p-phenylenediamine-type compound, the preferred examples of which include 4-amino-N,N-diethylaniline, 3-methyl-4-amino-N,N-diethylaniline, 4-amino-N-ethyl-N-β-hydroxyethylaniline, 3-methyl-4-amino-N-ethyl-N-β-hydroxyethylaniline, 3-methyl-4-amino-N-ethyl-N-β-methanesulfonamidoethylaniline, 3-methyl-4-amino-N-ethyl-N-β-methoxyethylaniline, 3-β-methanesulfonamidoethyl-4-amino-N,N-diethylaniline, 3-methoxy-4-amino-N-ethyl-N-β-hydroxyethylaniline, 3-methoxy-4-amino-N-ethyl-β-methoxyethylaniline, 3-acetamido-4-amino-N,N-diethylaniline, 4-amino-N,N-dimethylaniline, N-ethyl-N-β-[β-(β-methoxyethoxy)ethoxy]ethyl-3-methyl-4-aminoaniline, N-ethyl-N-β-(β-methoxyethoxy)ethyl-3-methyl-4-aminoaniline, and salts of these compounds such as sulfates, hydrochlorides, sulfites, p-toluenesulfates, and the like.

Further, there may be used those compounds as described in Japanese Patent O.P.I. Publication Nos.64932/1973, 131526/-1975 and 95849/1976, and Pent et al. the Journal of American Society Vol.73, pages 3100 - 3125 (1951), and the like. To these color developing agents may, if necessary, be added various additives such as alkali agent, pH control agent or buffer, development accelerator, antifoggant, preservative, and the like.

The color photographic light-sensitive material of the present invention, after exposed imagewise and subjected to

a color development, can be processed in a bleaching bath in usual manner. This processing may be either perfored concurrently with or separately from fixing. This bleaching bath, by adding, if necessary, a fixing agent thereto, can be used as a bleach-fix bath. As the bleaching agent, various compounds may be used together with a bleach accelerator and other additives.

The present invention will be illustrated further in detail with reference to examples below, but the invention is not limited thereto.

## EXAMPLE 1

A multilayer color negative light-sensitive material was prepared as Sample 1, which has the following construction (layer weight: $g/m^2$) comprising a support having thereon dye image-formable blue-sensitive, green-sensitive and red-sensitive silver halide emulsion layers.

Layer 1.....mordant layer:

| | |
|---|---|
| gelatin | 1.80 |
| mordant (the following Compound 1) | 1.00 |

Layer 2.....gelatin overcoat layer

Layer 3.....ultraviolet absorbing layer

Layer 4.....high-speed blue-sensitive emulsion layer:

| | |
|---|---|
| silver iodobromide (7 mole% silver iodide) | 2.20 |

| | |
|---|---|
| gelatin | 1.75 |
| Exemplified coupler (C-3) | 0.31 |
| Exemplified DIR Compound (D-18) | 0.06 |

Layer 5.....low-speed blue-sensitive emulsion layer:

| | |
|---|---|
| silver iodobromide (6 mole% silver iodide) | 1.36 |
| gelatin | 2.14 |
| yellow coupler (the following Compound 2) | 0.66 |

Layer 6.....yellow filter layer

Layer 7.....green-sensitive emulsion layer

Layer 8.....red-sensitive emulsion layer

Layer 9.....antihalation layer

Support.....cellulose triacetate film base

Sample 2 was subsequently prepared in quite the same manner as in Sample 1 with the exception that the Exemplified DIR compound (D-18) was excluded and the silver halide was reduced in the quantity to $1.20g/m^2$.

On the other hand, Sample 3 also was prepared in quite the same manner as in Sample 1 with the exception that an yellow coupler (the following Compound 2) was used in place of the Exemplified Coupler (C-3) of the high-speed blue-sensitive emulsion layer, and the mordant layer was excluded.

Sample 3' also was prepared in the same manner as in Sample 2 except that following yellow Compound 2 was used in place of Exemplified Coupler (C-3).

The above Exemplified Coupler (C-3) is a diffusible dye-producible coupler, while the coupler as the following Compound 2 and other couplers used in Samples 1 to 3 are all non-diffusible dye-producible couplers. The reason that the quantity of the silver halide was reduced in Sample 2 is because all the samples should be adjusted so as to indicate almost equal characteristic curves.

[Compound 1....mordant]

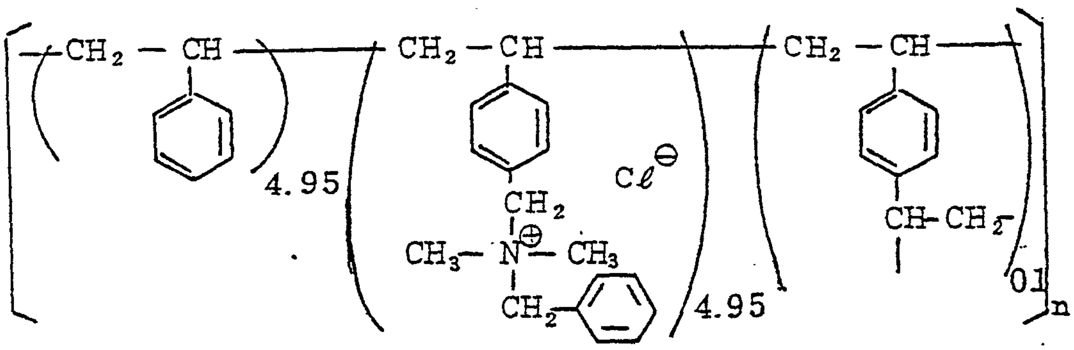

[Compound 2....yellow coupler]

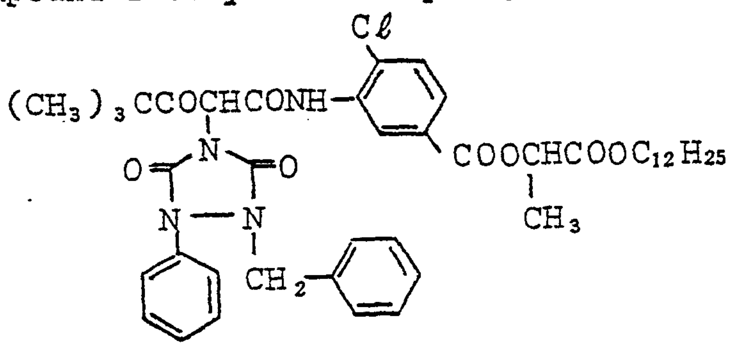

The thus obtained Samples 1 to 3 each was subjected to an wedge exposure in usual manner, and then processed in the following liquids in accordance with the development process steps below, thereby obtaining the results as given in Table 1. In addition, in regard to the red-sensitive and green-sensitive layers that showed substantially the same response in the present example, descriptions of their results are

omitted herefrom.

Development Process Steps:

| Steps (38°C) | Processing Time |
| --- | --- |
| Color developing | 3 min. 15 sec. |
| Bleaching | 6 min. 30 sec. |
| Washing | 3 min. 15 sec. |
| Fixing | 6 min. 30 sec. |
| Washing | 3 min. 15 sec. |
| Stabiliaing | 1 min. 30 sec. |

Compositions of the respective processing liquids used in the development process are as follows:

Color Developer Composition:

| | |
| --- | --- |
| 4-amino-3-methyl-N-ethyl-N-(β-hydroxyethyl)-aniline sulfate | 4.75g |
| Anhydrous sodium sulfite | 4.25g |
| Hydroxyamine 1/2 sulfate | 2.0g |
| Anhydrous sodium carbonate | 37.5g |
| Sodium bromide | 1.3g |
| Trisodium nitrilotriacetate, monohydrated | 2.5g |
| Potassium hydroxide | 1.0g |
| Water to make 1 liter | |
| Use potassium hydroxide to adjust the pH to 10.0 | |

Bleaching Bath Composition:

| | |
|---|---|
| Iron-ammonium ethylenediamine tetraacetate | 100.0g |
| Diammonium ethylenediamine tetraacetate | 10.0g |
| Ammonium bromide | 150.0g |
| Glacial acetic acid | 10.0ml |
| Water to make 1 liter | |
| Use aqueous ammonia to adjust the pH to 6.0 | |

Fixer Composition:

| | |
|---|---|
| Ammonium thiosulfate (aqueous 50% solution) | 162.0ml |
| Anhydrous sodium sulfite | 12.4g |
| Water to make 1 liter | |
| Use acetic acid to adjust the pH to 6.5. | |

Stabilizer Composition:

| | |
|---|---|
| Formalin (aqueous 37% solution) | 5.0ml |
| Koniducks (product of Konishiroku Photo Industry Co., Ltd.) | 7.5ml |
| Water to make 1 liter | |

Each of the samples processed in the above processing baths was measured for the granularity of the developed dye image thereof in a blue light by the RMS (Root Mean Square) method using a microdensitometer having a 25-micron diameter circular scanning head. And the measured results are given in Table 1 in the normalized granularities ($\delta N$) obtained at densities 1.2 and 2.3.

The normalized granularity can be obtained by first subtracting from the density of the masking colored coupler in the film the contribution to the above density, and then by dividing the RMS granularity by the subtracted density.

On the other hand, each of Samples 1 to 3 was exposed so that its density becomes 1.8 to soft X-rays through a wedge having spatial frequencies varied in the range of from 3 lines/-mm to 100 lines/mm, and processed in the same manner as previously described. The obtained color image was used to obtain MTF (Modulation Transfer Function) using a blue light. The resulting MTF values under the spatial frequency condition of 30 lines/mm are as given in Table 1. The MTF value is obtained by the density measurement made through a slitter with the dimensions of 300 mcrons by 2 microns, and shown in percentage of the output to the input.

In addition, the speed values given in Table 1 are each represented by the relative speed to the speed of Sample 1 regarded as 100.

Table 1

| Sample No. | Speed | $\gamma_2$ | Lowest density | Highest density | $\sigma N \times 1{,}000$ | | MTF 30 lines/mm |
|---|---|---|---|---|---|---|---|
| | | | | | D=1.2 | D=2.3 | |
| 1 | 100 | 0.64 | 0.81 | 3.15 | 35 | 25 | 83 |
| 2 | 102 | 0.63 | 0.83 | 3.17 | 43 | 27 | 74 |
| 3 | 98 | 0.66 | 0.80 | 3.21 | 47 | 29 | 85 |
| 3' | 101 | 0.63 | 0.83 | 3.16 | 50 | 30 | 82 |

0110633

As apparent from Table 1, although Samples 1 to 3 all provide almost equal characteristic curves, Sample 1 for the present invention is excellent in the sharpness as well as in the granularity as compared to Samples 2 and 3.

EXAMPLE 2

A light-sensitive material was prepared which is of the following construction comprising a support having thereon dye image-formable red-sensitive silver halide emulsion layers (layer weight: $g/m^2$). This was regarded as Sample 4.

Layer 1.....protective layer

Layer 2.....high-speed red-sensitive emulsion layer:

| | |
|---|---|
| silver iodobromide (4 mole% silver iodide) | 1.40 |
| gelatin | 1.95 |
| Exemplified Coupler (C-27) | 0.32 |

Layer 3.....low-speed red-sensitive emulsion layer:

| | |
|---|---|
| silver iodobromide (4 mole% silver iodide) | 1.82 |
| gelatin | 2.30 |
| coupler (the following Compound 3) | 0.59 |

Support.....cellulose triacetate film base

Sample 5 was subsequently prepared in quite the same manner as in Sample 4 with the exception that $0.04g/m^2$ of Exemplified DIR Compound (D-5) was used in the high-speed red-sensitive emulsion layer, and the silver iodobromide was increased in the quantity to $2.10g/m^2$.

Further, Sample 6 was also prepared in quite the same manner as in Sample 5 with the exception that $0.05g/m^2$ of Exemplified DIR Compound (D-10) was used in the low-speed red-sensitive emulsion layer, and the silver iodobromide was increased in the quantity to $2.90g/m^2$.

The reason that the quantity of the silver halide was increased as in above in Samples 5 and 6 is because of the need for controlling the samples, taking into account possible softening of the gradient due to the addition of the DIR compound, so that almost equal characteristic curves can be obtained.

[Compound 3....coupler]

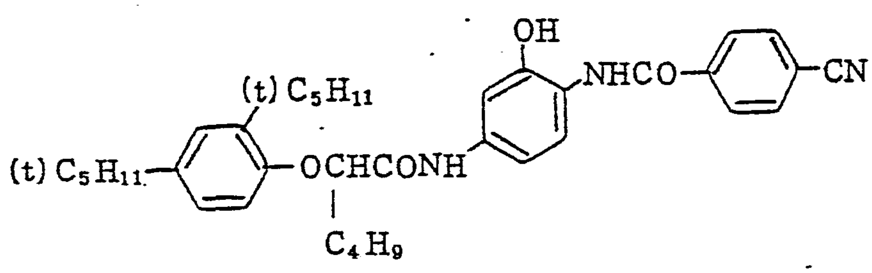

Each of the above-obtained Samples 4 to 6 was exposed through an optical wedge to light, and processed in the same manner as in Example 1. The obtained results are as shown in Table 2.

In the table, the normalized granularity is shown with the values in densities 0.7 and 1.7, and MFT is the value in the spatial frequency of 30 lines/mm obtained when each sample

is exposed to soft X-rays so that its density becomes 1.2.

Table 2

| Sample No. | Speed | $\gamma$ 2 | Minimum density | Maximum density | $\sigma$N x 1,000 | | MTF 30 lines/mm |
|---|---|---|---|---|---|---|---|
| | | | | | D=0.7 | D=1.7 | |
| 4 | 100 | 0.61 | 0.24 | 2.18 | 45 | 24 | 66 |
| 5 | 97 | 0.63 | 0.22 | 2.24 | 38 | 21 | 87 |
| 6 | 95 | 0.58 | 0.21 | 2.15 | 38 | 13 | 102 |

As apparent from the above table, Samples 4 to 6 all show almost equal characteristic curves. However, Sample 5 for the present invention, wherein the high-speed emulsion layer contains additionally the DIR compound, has the excellent effect for improving the sharpness as well as the granularity as compared to the comparative Sample 4. In Sample 6 for the present invention containing the DIR compound also in the low-speed emulsion layer thereof displays a remarkable effect for improving particularly the granularity and sharpness in the high exposure scale.

The above effects of the present invention is considered due to the increase in color forming points and the diffusing effect of the development inhibitor released from the DIR compound.

EXAMPLE 3

A multilayer color negative light-sensitive material was prepared by coating on a transparent polyethylene terephthalate support the following layers in the described order from the support side, and this was regarded as the sample for the present invention.

First layer...antihalation layer:

An aqueous gelatin solution containing black colloidal silver was coated so that the silver quantity is $0.3g/m^2$ and the dry thickness becomes 3.0μ.

Second layer...interlayer:

An aqueous gelatin solution was coated so that the dry thickness becomes 1.0μ.

Third layer...red-sensitive low-speed silver halide emulsion layer:

A silver iodobromide emulsion (a mixture of a silver iodobromide emulsion of mean particle size of 0.6μ containing 4 mole% of silver iodide with a silver iodobromide emulsion of mean particle size of 0.3μ containing 4 mole% of silver iodide in the proportion of 2 : 1) was chemically sensitized by the addition thereto of gold and sulfur sensitizers and further spectrally sensitized by the addition thereto of red-sensitizing dyes anhydrous 9-ethyl-3,3'-(3-sulfopropyl)-4,5,4',5'-dibenzothiacarbocyanine-hydroxide, anhydrous 5,5-dichloro-9-ethyl-3,3'-di-(3-sulfobutyl)thiacarbocyanine-hydroxide, and anhydrous 2-[2-{(5-chloro-3-ethyl-2(3H)-benzothiazolidene)methyl}]-1-butenyl-5-chloro-3-(4-sulfobutyl)benzoxazolium, and after that 1.0g of 4-hydroxy-6-methyl-1,3,3a,7-tetrazaindene

and 20.0mg of 1-phenyl-5-mercaptotetrazole, thereby preparing a red-sensitive low-speed emulsion.

To this emulsion was added a dispersed mixture liquid prepared in the manner that per mole of silver halide 59g of a cyan coupler 1-hydroxy-N-[$\delta$-(2,4-di-t-aminophenoxy)butyl]-2-naphthoamide, 4g of a colored cyan coupler disodium 1-hydroxy-4-[4-(1-hydroxy-8-acetamido-3,6-disulfo-2-naphthylazo)-phenoxy]-N-[$\delta$-(2,4-di-t-amylphenoxy)butyl]-2-naphthoamide, 6.2g of Exemplified Compound D-12 as a DIR compound, and 0.5g of dodecyl gallate were dissolved by heating into a mixture solvent of 65g of dibutyl phthalate with 136ml of ethyl acetate, and the resulting solution was added to 55ml of an aqueous 7.5% gelatin solution containing 5g of sodium triisopropylnaphthalenesulfonate, and the resulting mixture was emulsified to be dispersed by a colloid mill, whereby a red-sensitive low-speed emulsion (containing 160g of gelatin per mole of silver halide) was prepared, which was then coated so that the dry thickness becomes 40μ.

Fourth layer...red-sensitive high-speed silver halide emulsion layer:

A silver iodobromide emulsion (of mean particle size of 1.2μ, containing 7 mole% of silver iodide) was chemically sensitized by the addition thereto of gold and sulfur sensitizers, and further spectrally sensitized by the addition thereto of red-sensitizing dyes anhydrous 9-ethyl-3,3'-di-(3-sulfopropyl)-4,5,4', 5'-dibenzothiacarbocyanine-hydroxide, anhydrous 5,5'-dichloro-9-ethyl-3,3'-di-(3-sulfobutyl)thiacarbocyanine-hydroxide, and anhydrous 2-[2-{(5-chloro-3-ethyl-2(3H)-benzothiazolidene)methyl}-1-butenyl-5-chloro-3-(4-sulfobutyl)benzoxazolium, and after that 1.0g of 4-hydroxy-6-methyl-1,3,3a,7-tetrazaindene and 10.0ml of 1-phenyl-5-mercaptotetrazole were added, thereby preparing a red-sensitive high-speed emulsion.

To this emulsion was added a dispersed mixture liquid prepared in the manner that per mole of silver halide 28g of a cyan coupler Exemplified Coupler C-22, 12g of 1-hydroxy-4-isopropylaminocarbonylmethoxy-N-dodecyl-2-naphthoamide, 4g of a colored cyan coupler disodium 1-hydroxy-4-[4-(1-hydroxy-8-acetamido-3,6-disulfo-2-naphthylazo)phenoxy]-N-[$\delta$-(2,4-di-t-amylphenoxy)butyl]-2-naphthoamide, 3.7g of Exemplified DIR compound D-13, 0.5g of dodecyl gallate, and 0.5g of 2,5-di-t-octyl-hydroquinone were dissolved by heating into a mixute of 20g of dibutyl phthalate with 60ml of ethyl acetate, and this solution was added to 300ml of an aqueous 7.5% gelatin solution containing 1.5g of sodium triisopropyl-naphthalenesulfonate, and emulsified to be dispersed by means of a colloid mill, whereby a red-sensitive high-speed emulsion (containing 160g of gelatin per mole of silver halide) was prepared, which was then coated so that the dry thickness becomes 2.0μ.

Fifth layer...interlayer:

The same as the second layer.

Sixth layer...green-sensitive low-speed silver halide emulsion layer:

A silver iodobromide emulsion of mean particle size of 0.6μ containing 4 mole% of silver iodide and a silver iodobromide emulsion of mean particle size of 0.3μ containing 7 mole % of silver iodide each was chemically sensitized by the addition thereto of gold and sulfur sensitizers, and further spectrally sensitized by the addition thereto of green-sensitizing dyes anhydrous 5,5'-dichloro-9-ethyl-3,3'-di-(3-sulfobutyl)oxacarbocyanine-hydroxide, anhydrous 5,5'-diphenyl-9-ethyl-3,3-di-(3-sulfobutyl)oxacarbocyanin-hydroxide, and anhydrous 9-ethyl-3,3-di-(3-sulfopropyl)-5,6,5',6'-dibenzoxacarbocyanine-hydroxide, and then 1.0g of 4-hydroxy-6-methyl-1,3,3a,7-tetrazaindene and 20.0ml of 1-phenyl-5-mercaptotetrazole were added to thereby produce separately in usual manner two emulsions. The thus produced two emulsions were mixed in the proportion of 1 : 1, whereby a green-sensitive low-speed silver halide emulsion was prepared.

Further, to the resulting emulsion was added a dispersed mixture liquid prepared in the manner that per mole of silver halide 80g of a magenta coupler 1-(2,4,6-trichlorophenyl)-3-{3-(4-dodecyloxyphenyl)sulfonamidobenzamido}-pyrazoline-5-one, 35g of Exemplified Coupler C-18, 5.3g of Exemplified DIR Compound D-16 as a DIR compound, 2.5g of a colored magenta coupler 1-(2,4,6-trichlorophenyl)-4-(1-naphthylazo)-3-(2-chloro-5-octadecenylsuccinimidoanilino)-5-pyrazolone, and 0.5g of dodecyl gallate were dissolved by heating into a mixture of 120g of tricresyl phosphate and 240ml of ethyl acetate, and the resulting solution was added to an aqueous gelatin solution containing triisopropyl-naphthalenesulfonate, and emulsified to be dispersed by means of a colloid mill, whereby a green-sensitive low-speed emulsion (containing 160g of gelatin per mole of silver) was prepared, which was then coated so that the dry thickness becomes 4.0μ.

Seventh layer...green-sensitive high-speed silver halide emulsion layer:

A silver iodobromide emulsion (of mean particle size of 1.2μ, containing 7 mole% of silver iodide) was chemically sensitized by gold and sulfur sensitizers, and spectrally sensitized by the addition thereto of anhydrous 5,5'-dichloro-9-ethyl-3,3'-di-(3-sulfobutyl)oxacarbocyanine-hydroxide, anhydrous 5,5-diphenyl-9-ethyl-3,3'-di-(3-sulfobutyl)oxacarbocyanine-hydroxide and anhydrous 9-ethyl-3,3'-di-(3-sulfopropyl)-5,6,5',6'-dibenzoxacarbocyanine-hydroxide, and further, 1.0g of 4-hydroxy-6-methyl-1,3,3a,7-tetrazaindene and 10.0mg of 1-phenyl-5-mercaptotetrazole were added, thereby preparing a green-sensitive high-speed silver halide emulsion.

Further, to the emulsion was added a dispersed mixture liquid prepared in the manner that 52g of Exemplified Coupler C-18 as a magenta coupler, 30g of 1-(2,4,6-trichlorophenyl)-3-

{3-(2,4-di-t-amylphenoxyacetamido)benzamido} pyrazoline-5-one, 2.5g of a colored magenta coupler 1-(2,4,6-trichlorophenyl)-4-(1-naphthylazo)-3-(2-chloro-5-octadecylsuccinimidoanilino)-5-pyrazolone, 3.4g of Exemplified Compound D-6, and 15g of 2,5-di-t-octyl-hydroquinone were dissolved by heating into a mixture of 120g of tricresyl phosphate and 240ml of ethyl acetate, and the solution was added to an aqueous gelatin solution containing sodium triisopropyl-naphthalenesulfonate, and emulsified to be dispersed by means of a colloid mill, whereby a green-sensitive high-speed emulsion (containing 160g of gelatin per mole of silver halide), which was then coated so that the dry thickness becomes 2.0μ.

Eighth layer...interlayer:

The same as the second layer.

Ninth layer...yellow filter layer:

To an yellow colloidal silver-dispersed aqueous gelatin solution was added a dispersed liquid that was prepared in the manner that 3g of 2,5-di-t-octyl-hydroquinone and 1.5g of di-2-ethyl-hexyl phthalate were dissolved into 10ml of ethyl acetate, and this solution was dispersed into an aqueous gelatin solution containing 0.3g of sodium triisopropyl-naphthalenesulfonate. The resulting mixture liquid was coated so that the coating quantity of the gelatin is $0.9g/m^2$, that of the 2,5-di-t-octyl-hydroquinone is $0.10g/m^2$, and the dry thickness becomes 1.2μ.

Tenth layer... blue-sensitive low-speed silver halide emulsion layer:

A silver iodobromide emulsion (of mean particle size of 0.6μ, containing 6 mole% of silver iodide) was chemically sensitized by gold and sulfur sensitizers, and spectrally sensitized by the addition thereto of a blue-sensitizing dye anhydrous 5,5'-dimethoxy-3,3'-di-(3-sulfopropyl)thiacyanine-hydroxide, and then 1.0g of 4-hydroxy-6-methyl-1,3,3a,7-tetrazaindene and 20.0mg of 1-phenyl-5-mercaptotetrazole were added to thereby prepare in usual manner a blue-sensitive low-speed silver halide emulsion.

Further, to the emulsion was added a dispersed mixture liquid that was prepared in the manner that per mole of silver halide as yellow couplers 120g of α-pivaloyl-α-(1-benzyl-2-phenyl-3,5-dioxo-1,2,4-triazolidine-4-yl)-2-chloro-5'-[α-(dodecyloxycarbonyl)ethoxycarbonyl]acetanilide and 50g of α-{3-[α-(2,4-di-t-amylphenoxy)butylamido}benzoyl -2'-methoxyacetanilide, and 15g of Exemplified Compound C-6 as a DIR compound were dissolved by heating into a mixture of 120g of dibutyl phthalate and 300ml of ethyl acetate, and this solution was added to an aqueous gelatin solution containing sodium triisopropyl-naphthalenesulfonate and emulsified to be dispersed by means of a colloid mill, whereby a blue-sensitive low-speed silver halide emulsion (containing 160g of gelatin per mole of silver halide) was prepared, which was then coated so that

the dry thickness becomes 4.0μ.

Eleventh layer...blue-sensitive high-speed silver halide emulsion layer:

A silver iodobromide emulsion (of mean particle size of 1.2μ, containing 7 mole% of silver iodide) was chemically sensitized by gold and sulfur sensitizers, and spectrally sensitized by the addition thereto of a blue-sensitizing dye anhydrous 5,5'-dimethoxy-3,3'-di-(3-sulfopropyl)thiacyanine-hydroxide, and then 1.0g of 4-hydroxy-6-methyl-1,3,3a,7-tetrazaindene and 10.0mg of 1-phenyl-5-mercaptotetrazole were added to thereby prepare in usual manner a blue-sensitive high-speed silver halide emulsion.

Further, to the resulting emulsion was added a dispersed mixture liquid that was prepared in the manner that per mole of silver halide 80g of Exemplified Coupler C-14 as an yellow coupler and 7.4g of Exemplified DIR Compound D-6 were dissolved by heating into a mixture of 80g of dibutyl phthalate with 240 ml of ethyl acetate, and this solution was added to an aqueous gelatin solution containing sodium triisopropyl-naphthalenesulfonate, and emulsified to be dispersed by means of a colloid mill, whereby a blue-sensitive high-speed silver halide emulsion (containing 240g of gelatin per mole of silver halide) was prepared, which was then coated so that the dry thickness becomes 2.0μ.

Twelfth layer....interlayer:

Two grams of di-2-ethyl-hexyl phthalate, 2g of 2-[3-cyano-3-(n-dodecylaminocarbonyl)arylidene]-1-ethylpyrolidine and 2ml of ethyl acetate were mixed, and the mixture was dispersed into an aqueous gelatin solution containing 0.6g of sodium triisopropyl-naphthalenesulfonate, and this was coated so that the coating quantity of the gelain is 1.0g/$m^2$, and the dry thickness becomes 1.0µ.

Thirteenth layer...protective layer:

An aqueous gelatin solution containing per mole 100ml 4g of gelatin and 0.2g of 1,2-bisvinyl-sulfonyl-ethane was coated so that the coating quantity of the gelatin is 1.3g/$m^2$ and the dry thickness becomes 1.2µ.

The thus obtained sample according to the present invention was slit into 35mm size film to be photographed and processed, thereby obtaining negative film images, from which were made enlarged color photographic prints. The color prints showed very fine and sharp details from the shadow to the highlight with bright and clear color reproductions, particularly excellent in the green and red color reproductions.

Thus, the present invention provides not only improvements on the raininess and sharpness but also a very excellent effect for color reproductions.

CLAIMS

1. A silver halide photographic light-sensitive material comprising a support having thereon a plurality of silver halide emulsion layers which possess different speeds but sensitivities in the same spectral region and at least one light-sensitive layer comprising a non-diffusible coupler capable of forming non-diffusible dye by reaction with an oxidation product of a color developing agent, wherein the silver halide emulsion layer having the highest speed among said plurality of silver halide emulsion layers contains a non-diffusible coupler capable of producing a movable dye by reaction thereof with an oxidation product of a color developing agent and a non-diffusible compound capable of releasing a development inhibitor by reaction thereof with an oxidation product of a color developing agent.

2. A silver halide photographic light-sensitive material according to claim 1 which comprises, in order on the support, at least one red-sensitive layer, green-sensitive layer and blue-sensitive layer.

3. A silver halide photographic light-sensitive material according to claim 2, wherein one of the red-sensitive layer, the green-sensitive layer and the blue-sensitive layer comprises a plurality of silver halide emulsion layers whose speeds are different from each other.

4. A silver halide photographic light-sensitive material according to claim 2, wherein the red-sensitive layer, the green-sensitive layer and the blue-sensitive layer each comprise a plurality of silver halide emulsion layers whose speeds are different from each other.

5. A silver halide photographic light-sensitive material according to claim 3 or 4, wherein of the said plurality of silver halide emulsion layers whose speeds are different, the layer closer or closest to the support has a low speed.

6. A silver halide photographic light-sensitive material according to any one of claims 1 to 5, wherein the non-diffusible coupler capable of producing the movable dye is a coupler capable of producing a dye capable of moving in the layer containing the non-diffusible coupler.

7. A silver halide photographic light-sensitive material according to any one of claims 1 to 5, wherein the movable dye-producible non-diffusible coupler is a coupler capable of producing a dye capable of moving and diffusing from the layer containing the non-diffusible coupler.

8. A silver halide photographic light-sensitive material according to any one of claims 1 to 7, wherein the non-diffusible coupler capable of producing the movable dye has the formula:

```
COUP ——— control group        (I)
|
stabilizing group
```

wherein COUP represents a coupler component for producing a dye, the "stabilizing group" is a group that is linked to the above coupler component in a coupling position thereof and which can be split from COUP during a coupling reaction between the coupler and an oxidation product of a color developing agent, the stabilizing group having a molecular size sufficient to render the coupler non-diffusible, and the "control group" is a group that is linked to COUP in a non-coupling position thereof and which controls the dye produced by the coupling reaction of the coupler with an oxidation product of a color developing agent so as to render said dye slightly movable or completely diffusible.

9. A silver halide photographic light-sensitive material according to claim 8, wherein the stabilizing group in Formula (I) is a group having an alkyl component or an aryl component each having not less than eight carbon atoms.

10. A silver halide photographic light-sensitive material according to claim 9, wherein the stabilizing group in Formula (I) is one having an alkyl component or an aryl component, each having 8 to 32 carbon atoms.

11. A silver halide photographic light-sensitive material according to claim 10, wherein the stabilizing group of Formula (I) is an alkoxy group, an aryloxy group, an alkylthio group, an arylthio group or a nitrogen-containing heterocyclic group, each having 8 to 32 carbon atoms.

12. A silver halide photographic light-sensitive material according to any one of claims 8 to 11, wherein the control group in Formula (I) is an alkyl group having 1 to 20 carbon atoms or an aryl group having 6 to 20 carbon atoms.

13. A silver halide photographic light-sensitive material according to any one of claims 8 to 11, wherein the control group in Formula (I) is an alkali-soluble group capable of being ionized.

14. A silver halide photographic light-sensitive material according to any one of claims 8 to 13, wherein the non-diffusible coupler has the formula:

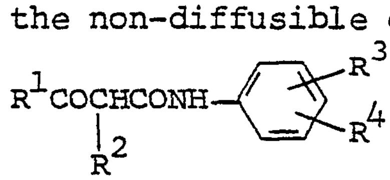

(II)

wherein $R^1$ is an aryl group or an alkyl group; $R^2$ is a said stabilizing group; $R^3$ is a said control group; and $R^4$ represents a hydrogen atom, a halogen atom or an alkyl or alkoxy group having 1 to 8 carbon atoms, or a control group.

15. A silver halide photographic light-sensitive material according to any one of claims 8 to 13, wherein the non-diffusible coupler has the formula:

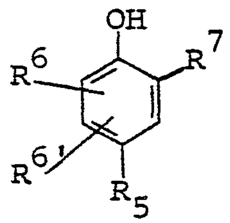 (III) or 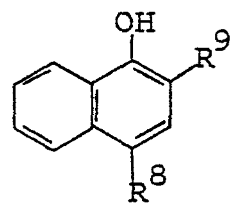 (IV)

wherein $R^5$ represents a group as defined under $R^2$ in claim 15; one of $R^6$, $R^{6'}$ or $R^7$ represents a said control group and the other two are the same or different and represent a hydrogen atom, a halogen atom, an alkyl group, an alkoxy group, an alkylamino group, or an acylamido group each having 1 to 8 carbon atoms; and $R^8$ is a group as defined under $R^5$; and $R^9$ represents a control group.

16. A silver halide photographic light-sensitive material according to any one of claims 8 to 13, wherein the non-diffusible coupler has the formula:

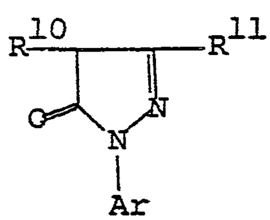 (V) or 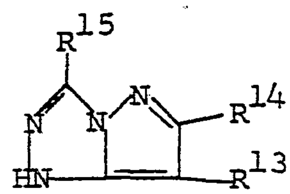 (VI)

wherein $R^{10}$ represents a group as defined under $R^5$ in claim 15; $R^{11}$ represents a control group; Ar is a phenyl group which is optionally substituted by at least one of a hydrogen atom, an alkyl group, an alkoxy group or an amino group, the phenyl group being capable of being said control group; $R^{13}$ is a group as defined under $R^{10}$; and one of $R^{14}$ and $R^{15}$ is a control group and the other is a hydrogen atom, an alkyl group, an alkoxy group having 1 to 8 carbon atoms, an aryl group having 6 to 10 carbon atoms, or an amino or acylamino group.

17. A silver halide photographic light-sensitive material according to any one of claims 1 to 16, wherein the non-diffusible compound capable of

releasing a development inhibitor has the formula:

COUP - inhibitor (VII)

wherein COUP represents a coupler component capable of coupling with an oxidization product of a color developing agent; and the "inhibitor" is a compound that inhibits the development of silver halides,

or COUP - TIME - inhibitor (VIII)

wherein "inhibitor" is as defined above; COUP is as defined above; and TIME represents a group having the formula:

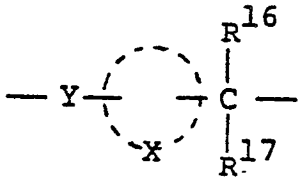

(IX)

wherein X is a group of atoms necessary to complete a benzene or naphthalene ring; Y is -O-, -S-, or

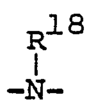

(wherein $R^{18}$ is a hydrogen atom, an alkyl group or an aryl group), any of which may be linked to the ring in a coupling position thereof; and $R^{16}$ and $R^{17}$ each independently is as defined under $R^{18}$, but the group

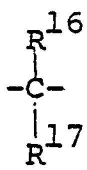

is substituted in the ortho or para position to Y and bonded to a hetero atom contained in the inhibitor,

or

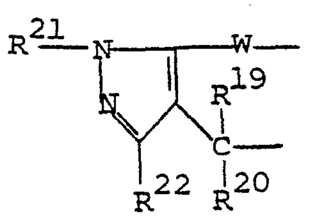

(X)

wherein W is a group as defined under Y above; $R^{19}$ and $R^{20}$ are as defined under $R^{16}$ and $R^{17}$ above; $R^{21}$ is a hydrogen atom, an alkyl group, an aryl group, an acyl group, a sulfone group, an alkoxycarbonyl group or a heterocyclic residue; and $R^{22}$ is a hydrogen atom, an alkyl group, an aryl group, a heterocyclic residue, an alkoxy group, an amino group, an acylamido group, a sulfonamido group, a carboxy group, an alkoxycarbonyl group, a carbamoyl group, or a cyano group, and the said TIME group is linked by W to COUP in a coupling position thereof and bonded by the

$$-\overset{R^{19}}{\underset{R^{20}}{\overset{|}{\underset{|}{C}}}}-$$

group to a hetero atom of the inhibitor,

```
|
Nu          (XI)
|
V - E -
```

wherein Nu is an electron-rich nucleophilic group containing an oxygen, sulfur or nitrogen atom and is linked to COUP in a coupling position thereof; E is an electron-poor electrophilic group containing a carbonyl, thiacarbonyl, phosphonyl or thiophosphinyl group and is bonded to a hetero atom of the inhibitor; and V is a linkage group which establishes the steric relation of Nu with E, and which, after Nu is released from COUP, is capable of being subjected to an intramolecular nucleophilic substitution reaction accompanied by the formation of a 3- to 7-member cyclic ring, and is thereby capable of releasing an inhibitor.

18. A silver halide photographic light-sensitive material according to claim 17, wherein the inhibitor is benzotriazole, 3-octylthio-1,2,4-triazole or a heterocyclic mercapto compound.

19. A silver halide photographic light-sensitive material according to any one of the preceding claims, wherein the movable dye-producible

non-diffusible coupler is present in the highest-speed silver halide emulsion layer in an amount from $2x10^{-3}$ moles to $2x10^{-1}$ moles per mole of silver.

20. A silver halide photographic light-sensitive material according to any one of the preceding claims, wherein the non-diffusible compound capable of releasing the development inhibitor is present in the highest-speed silver halide emulsion layer in an amount from $10^{-3}$ moles to $10^{-1}$ moles per mole of silver.

21. A silver halide photographic light-sensitive material according to any one of the preceding claims, wherein the non-diffusible color dye-producible non-diffusible coupler is present in an amount from 1/50 to 10 times the amount of the movable dye-producible non-diffusible coupler.

22. A silver halide photographic light-sensitive material according to any one of the preceding claims, wherein the highest-speed silver halide emulsion layer contains a mordant.

23. A silver halide photographic light-sensitive material according to claim 22, wherein the mordant is present in an amount from 0.1 to 5 $g/m^2$.

24. A silver halide photographic light-sensitive material according to claim 23, wherein the mordant is present in an amount from 0.3 to $1.5g/m^2$.

25. A silver halide photographic light-sensitive material according to any one of the preceding claims, wherein the support has thereon a plurality of the light-sensitive layers and a mordant layer.

26. A silver halide photographic light-sensitive material according to any one of the preceding claims, wherein the highest-speed silver halide emulsion layer also contains a non-diffusible dye-producible non-diffusible coupler, and a silver halide emulsion layer having a lower speed than that of the above highest-speed emulsion layer contains a movable dye-producible

non-diffusible coupler and a DIR compound.

27. A silver halide photographic light-sensitive material according to any one of claims 1 to 25, wherein the highest-speed silver halide emulsion layer also contains a non-diffusible dye-producible non-diffusible coupler, and a silver halide emulsion layer having a lower speed than that of the above emulsion layer contains a non-diffusible dye-producible non-diffusible coupler and a DIR compound.

28. A silver halide photographic light-sensitive material according to any one of claims 1 to 25, wherein a silver halide emulsion layer having a lower speed than that of the highest speed emulsion layer contains a movable dye-producible non-diffusible coupler, a non-diffusible dye-producible non-diffusible coupler and a DIR compound.

29. A silver halide photographic light-sensitive material according to any one of claims 1 to 25, wherein a silver halide emulsion layer having a lower speed than that of the highest-speed emulsion layer contains a non-diffusible dye-producible non-diffusible coupler and a DIR compound.